# EUROPEAN PATENT APPLICATION

(11) **EP 4 257 666 A1**
(43) Date of publication of application: **11.10.2023**
(21) Application number: 21900244.1
(22) Date of filing: 25.02.2021
(51) Int. Cl.: C12M 1/00, C12M 1/34, C12N 15/12, C12N 15/31, C12N 15/52, C12Q 1/686

(54) **NUCLEIC ACID AMPLIFICATION METHOD AND THERMAL CYCLER**

(30) Priority: 04.12.2020 JP 2020202035
(71) Applicant: Yamaguchi University, Yamaguchi 753-8511 (JP); Yanagiya Machinery Co., Ltd., Ube-shi Yamaguchi, 759-0134 (JP)
(72) Inventor: FUJIMOTO Tetsunori, Ube-shi Yamaguchi 759-0134 (JP); NAKATA Noriatsu, Ube-shi Yamaguchi 759-0134 (JP); NAKATOMI Tadashi, Ube-shi Yamaguchi 759-0134 (JP); AKADA Rinji, Ube-shi, Yamaguchi 755-8611 (JP)
(74) Representative: Hasegawa, Kan
(86) International application number: PCT/JP2021/007148
(87) International publication number: WO 2022/118487

(57) **Abstract**

An object of the present invention is to provide a method for amplifying a nucleic acid using a PCR reaction solution with a volume as large as 30 mL or more, and an apparatus for amplifying a nucleic acid using a PCR reaction solution with a volume as large as 30 mL or more. The present invention provides a method for amplifying a nucleic acid, comprising performing polymerase chain reaction (PCR) by controlling a temperature of one or more reaction container(s) housing a reaction solution containing a DNA polymerase, deoxyribonucleotide triphosphates (dNTPs), a template DNA, a forward primer, a reverse primer, and a buffer solution, wherein the reaction solution is in an amount of 30 mL or more, and the polymerase chain reaction is performed while the reaction solution is stirred.

## Description

### Technical Field

The present invention relates to a method for amplifying a nucleic acid by performing polymerase chain reaction (PCR), and a thermal cycler.

### Background Art

In the analysis or analytics of nucleic acids, nucleic acids amplification approaches based on the replication of targeted nucleic acids are widely employed using polymerase chain reaction (PCR) method (see patent document 1). Such PCR is widely employed in the diagnosis of diseases, nucleic acid drugs, microbial analytics, research on genetic engineering, DNA evidence, etc., because its process is simple or the PCR can be performed in a relatively short time using an apparatus having a small and simple mechanism, for example.

Typical PCR efficiently amplifies DNA by performing 25 to 40 repetitive thermal cycles involving a series of steps of (1) the thermal denaturation of double-stranded template DNA, (2) the annealing of primers, and (3) extension mediated by DNA polymerase. For PCR, it is important to control the temperature of each step, and various techniques have been developed. For example, disclosed are: a nucleic acid amplification method comprising the step of adjusting the temperature of a reaction mixture in each sample vessel using a Peltier element as a cooling factor that comes into thermal contact with a liquid, wherein the liquid is stirred with a stirring bar (see patent document 2); and a temperature control apparatus for a microchip having a microchannel, the apparatus comprising a Peltier element having first and second faces opposed to each other, wherein the temperature of the microchip is controlled by driving a radiator fan during periods of microchip heating and cooling so as to achieve rapid temperature rise and drop operations and so as not to cause temperature variations on a heated face of the Peltier element (see patent document 3).

For PCR, it has previously been common to replicate a nucleic acid in several ng to several hundreds of ng in a PCR reaction solution in µL. However, the need of replicating DNA or RNA in a large amount has come with the development of vaccines, etc. in recent years. In the case of replication in a large amount by PCR, conventional techniques need to repeat PCR several tens to several hundreds of times and require labor, time and cost.

Under these circumstances, apparatuses for performing large-volume PCR have also been developed. For example, a thermal cycler comprising a pair of temperature regulation units is disclosed, wherein the temperature regulation units are symmetrically disposed above and below a sac-like member containing a reaction solution, and connected via connecting means so as to be slidable up and down (see patent document 4). This invention employs a polypropylene sac-like member and is configured such that, even when a container is in an inflated state by the reaction solution enclosed in the inside of the sac-like member, the distance between a pair of metal plates is regulated. Also, a Peltier element is used as the temperature regulation units.

Also disclosed are: a method for performing emulsion PCR by adding a PCR reaction solution into a flexible bag and bringing the flexible bag into contact with opposing faces of a thermal cycler (see patent document 5); and a method for amplifying a nucleic acid in a cuvette, wherein the cuvette may be rocked in order to mix components of a reaction solution contained in the cuvette (see patent document 6).

### Prior Art Documents

### Patent Documents

[Patent Document 1] Japanese Patent Publication No. 4-67957
[Patent Document 2] Japanese unexamined Patent Application Publication (Translation of PCT Application) No. 2009-537152
[Patent Document 3] Japanese unexamined Patent Application Publication No. 2017-063778
[Patent Document 4] Japanese unexamined Patent Application Publication No. 2018-139505
[Patent Document 5] U.S. Patent Publication No. 2010/0261230
[Patent Document 6] U.S. Patent No. 5229297

### Summary of the Invention

### Object to be Solved by the Invention

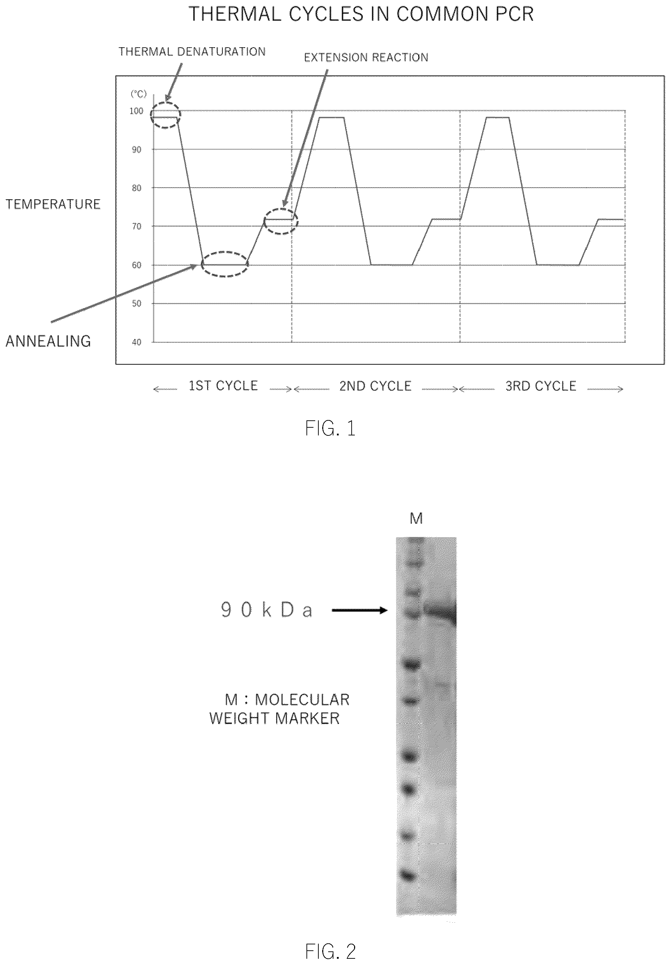

For amplifying a nucleic acid while maintaining specificity in PCR reaction, it is necessary to accurately control the temperature of a reaction container in each step for thermal cycles shown in Figure 1. Nonetheless, if a reaction solution has a large volume of 30 mL or more, a long time is required for temperature increase or decrease until the temperature reaches each set temperature for PCR thermal cycles. A small volume enables cycles in a short time, whereas a large volume requires conditions under which accurate DNA synthesis is accomplished by enduring temperature unevenness as well as cycles involving temperature increase and decrease for a long time. Conventional PCR reaction is commonly performed in 50 µL probably because a combination of a reaction buffer and DNA synthetase which accomplishes DNA synthesis by accurate PCR while enduring temperature control for a long time, and temperature evenness cannot be achieved. Accordingly, an object of the present invention is to provide a method for amplifying a nucleic acid using a PCR reaction solution with a volume as large as 30 mL or more, and an apparatus for amplifying a nucleic acid using a PCR reaction solution with a volume as large as 30 mL or more.

### Means to Solve the Object

The present inventors have conducted diligent studies to attain the object, and consequently completed the present invention by finding that, even in the case of using a PCR reaction solution with a volume as large as 30 mL or more, a nucleic acid can be specifically amplified by contriving the composition of the PCR reaction solution and stirring the reaction solution.

Specifically, the present invention is as follows.
[1] A method for amplifying a nucleic acid, comprising performing polymerase chain reaction (PCR) by controlling a temperature of one or more reaction container(s) housing a reaction solution containing a DNA polymerase, deoxyribonucleotide triphosphates (dNTPs), a template DNA, a forward primer, a reverse primer, and a buffer solution, wherein the reaction solution is in an amount of 30 mL or more, and the polymerase chain reaction is performed while the reaction solution is stirred.
[2] The method according to the above [1], wherein a concentration of the buffer solution is 0.4 to 1.5 M.
[3] The method according to the above [1] or [2], wherein pH of the reaction solution is 8.5 to 10.
[4] The method according to any one of the above [1] to [3], wherein the reaction solution is stirred by oscillating the reaction container.
[5] The method according to any one of the above [1] to [4], wherein the reaction container is made of a stretchable material.
[6] The method according to any one of the above [1] to [5], wherein 1 to 90% of a gas is contained in the reaction container.
[7] The method according to any one of the above [1] to [6], wherein an outside surface temperature of the reaction container or a temperature of the reaction solution is measured with a temperature sensor, and temperature control of thermal cycles in the polymerase chain reaction is performed based on the measured temperature.
[8] The method according to the above [7], wherein a surface temperature of a heat source adhering to the reaction container is measured with a temperature sensor, and the temperature control of the thermal cycles in the polymerase chain reaction is performed based on the outside surface temperature of the reaction container or the temperature of the reaction solution, and the surface temperature of the heat source adhering to the reaction container.
[9] A thermal cycler for use in the method of amplifying nucleic acids according to any one of the above [1] to [8], wherein the thermal cycler comprising: a temperature regulation unit which adheres to one or more reaction container(s) housing 30 mL or more of a reaction solution and regulates a temperature of the reaction solution, wherein the reaction container is made of a heat conductive material; and a stirring unit for stirring the reaction solution, wherein the temperature regulation unit comprises a heat source, a reaction container support plate which supports the reaction container by abutting an upper face of the reaction container, and a temperature sensor which measures an outside surface temperature of the reaction container or a temperature of the reaction solution.
[10] The thermal cycler according to the above [9], wherein the heat source in the temperature regulation unit is a Peltier element, and the thermal cycler comprises a heat conductive plate which adheres to the Peltier element and is capable of abutting the reaction container, and a temperature control part connected to the Peltier element.
[11] The thermal cycler according to the above [9] or [10], wherein temperature control of thermal cycles in polymerase chain reaction is performed based on the outside surface temperature of the reaction container or the temperature of the reaction solution measured with the temperature sensor.
[12] The thermal cycler according to any one of the above [9] to [11], wherein: a heat conductive plate is provided with a depression or a through-hole; a thermometry part of the temperature sensor is disposed in the depression or the through-hole without coming into contact with the heat conductive plate; and when the reaction container is placed on the heat conductive plate, the thermometry part of the temperature sensor is capable of abutting an underside of the reaction container.
[13] The thermal cycler according to any one of the above [9] to [12], wherein the thermal cycler comprises a temperature regulation unit for thermal denaturation, a temperature regulation unit for anneal and extension reaction, and a preheating or cooling temperature regulation unit.

### Effect of the Invention

The method for amplifying a nucleic acid according to the present invention enables a nucleic acid to be amplified even using a PCR reaction solution with a volume as large as 30 mL or more. The thermal cycler of the present invention enables the temperature of a reaction container or a reaction solution to be controlled such that a nucleic acid is amplified even using a PCR reaction solution with a volume as large as 30 mL or more.

### Brief Description of Drawings

[Figure 1] Figure 1 is a diagram showing an image of thermal cycles in common PCR.
[Figure 2] Figure 2 is a diagram showing results of analyzing a DNA polymerase Pfu solution by SDS-PAGE in Example 1.
[Figure 3] Figure 3 is a diagram showing results of agarose electrophoresis of a PCR product in Example 2.
[Figure 4] Figure 4 is a diagram showing results of agarose electrophoresis of a PCR product in Example 3.
[Figure 5] Figure 5 is a diagram showing results of agarose electrophoresis of a PCR product in Example 4.
[Figure 6] Figure 6 is a diagram showing results of agarose electrophoresis of a PCR product in Example 5.
[Figure 7] Figure 7 is a diagram showing results of agarose electrophoresis of a PCR product in Example 6.
[Figure 8A] Figure 8A is a schematic view of a temperature regulation unit used in Example 7.
[Figure 8B] Figure 8B is a diagram showing the arrangement of autoclave bags, Peltier elements, and aluminum plates placed in the thermal cycler of Figure 8A.
[Figure 8C] Figure 8(c) is a plane view of a state where a 200 µL reaction solution bag is disposed on the autoclave bag of the lower layer.
[Figure 9] The upper part of Figure 9 is a diagram showing results of agarose electrophoresis of a PCR product in Example 7. The lower part of Figure 9 is a diagram showing results of examining the temperatures of PCR thermal cycles for the 200 µL reaction solution bag at position 2 shown in Figure 8C.
[Figure 10A] Figure 10A is an underside perspective view of a state where a temperature sensor is affixed to the center of the underside of a reaction solution bag in Example 8.
[Figure 10B] Figure 10B is a top perspective view of a state where a thermometry part of a temperature sensor is disposed in a through-hole provided in an aluminum plate.
[Figure 11A] The upper part of Figure 11A is a diagram showing results of agarose electrophoresis of a PCR product in the case of using 100 mL of a reaction solution in Example 8. The lower part Figure 11A shows results of examining PCR thermal cycles in the case of using 100 mL of a reaction solution.
[Figure 11B] The upper part of Figure 11B is a diagram showing results of agarose electrophoresis of a PCR product in the case of using 200 mL of a reaction solution in Example 8. The lower part Figure 11B shows results of examining PCR thermal cycles in the case of using 200 mL of a reaction solution.
[Figure 11C] The upper part of Figure 11C(a) is a diagram showing results of agarose electrophoresis of a PCR product of a model in the case of using 300 mL of a reaction solution in Example 8. The lower part Figure 11C shows results of examining PCR thermal cycles of a model in the case of using 300 mL of a reaction solution.
[Figure 11D] Figure 11D shows results of examining PCR thermal cycles with no shake as a control in Example 8.
[Figure 12] Figure 12 shows results of examining PCR thermal cycles in the case of performing shake by different shake methods in Example 9.

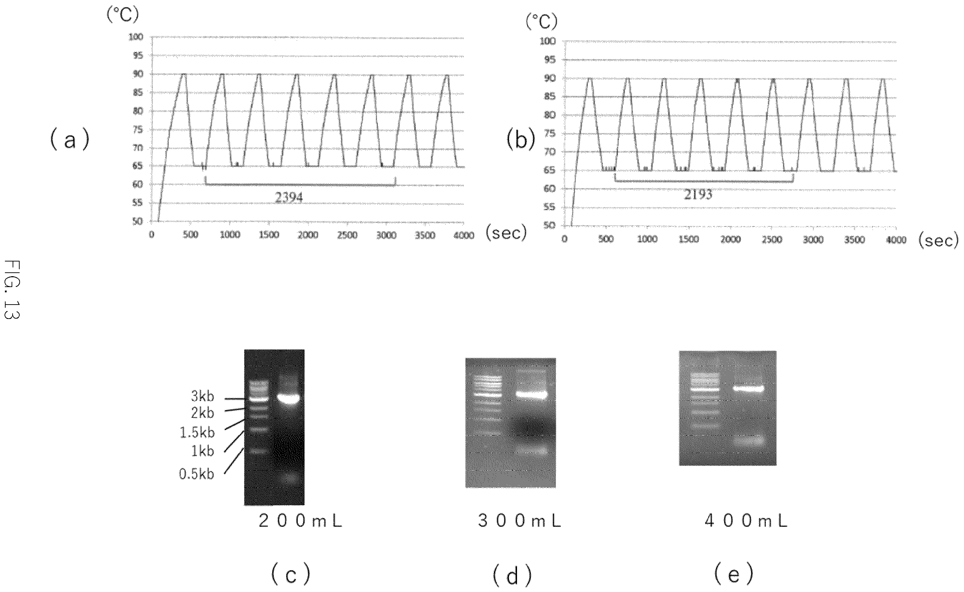

[Figure 13] The upper left part of Figure 13 shows results of examining PCR thermal cycles in the case of performing PCR by temperature control only on the lower side in Example 10. The upper right part of Figure 13 shows results of examining PCR thermal cycles in the case of performing PCR by temperature control both on the upper side and on the lower side. The bottom left of Figure 13 shows results of examining PCR thermal cycles in the case of performing PCR with a model of 200 mL of a reaction solution. The bottom center of Figure 13 shows results of examining PCR thermal cycles in the case of performing PCR with a model of 300 mL of a reaction solution. The bottom right of Figure 13 shows results of examining PCR thermal cycles in the case of performing PCR with a model of 400 mL of a reaction solution.
[Figure 14] The upper part of Figure 14 shows results of agarose electrophoresis of PCR products with a model of 600 mL of a reaction solution in Example 11. The lower part of Figure 14 shows results of examining PCR thermal cycles with a model of 600 mL of a reaction solution in Example 11.
[Figure 15] The upper part of Figure 15 shows results of agarose electrophoresis of PCR products with a model of 1000 mL of a reaction solution in Example 11. The lower part of Figure 15 shows results of examining PCR thermal cycles with a model of 1000 mL of a reaction solution in Example 11.
[Figure 16] Figure 16 is a front view of the whole of a first example of a thermal cycler.
[Figure 17A] Figure 17A is a front view of a temperature regulation unit in the first example of the thermal cycler.
[Figure 17B] Figure 17B is a right side view of the temperature regulation unit in this thermal cycler.
[Figure 18] Figure 18 is a plane view of a copper plate in the first example of the thermal cycler.
[Figure 19A] Figure 19A is a diagram showing the positions of a copper plate and a temperature sensor in the first example of the thermal cycler.
[Figure 19B] Figure 19B is a plane view of a state where a reaction container in which the temperature sensor is affixed onto the copper plate is placed in this thermal cycler.
[Figure 20A] Figure 20A is a front view of a reaction container support plate.
[Figure 20B] Figure 20B is a plane view of the reaction container support plate.
[Figure 20C] Figure 20C is a side view of the reaction container support plate.
[Figure 21A] Figure 21A is a diagram showing a state where a reaction container is swollen due to water vapor generated therein.
[Figure 21B] Figure 21B is a diagram showing the reaction container support plate fixed so as to provide a gap between the reaction container and the reaction container support plate.
[Figure 22A] Figure 22A is a front view of a temperature control unit in the second example of the thermal cycler.
[Figure 22B] Figure 22B is a plane view of a reaction container support plate of a second example of a thermal cycler.
[Figure 23] Figure 23 is a front view of a third example of a thermal cycler.
[Figure 24A] Figure 24A is a diagram showing the positional relationship between Peltier elements and a copper plate witch have a 1 cm diameter through-hole for installing a temperature sensor at the center in a form using eight Peltier elements are used in the lower face of a thermal cycler.
[Figure 24B] Figure 24B is a diagram showing the positional relationship between Peltier elements and a copper plate which have a length of 3 cm and width of 3 cm hollow in the center and a 1 cm diameter through-hole near the edge for installing a temperature sensor in a form using eight Peltier elements are used in the lower face of a thermal cycler.
[Figure 25] Figure 25 is a front view of a temperature regulation unit in the case of using a temperature probe as a temperature sensor.

### Mode of Carrying Out the Invention

The method for amplifying a nucleic acid according to the present invention is a method for amplifying a nucleic acid, comprising performing polymerase chain reaction (PCR) by controlling a temperature of one or more reaction container(s) housing a reaction solution containing a DNA polymerase, deoxyribonucleotide triphosphates (dNTPs), a template DNA, a forward primer, a reverse primer, and a buffer solution, wherein the reaction solution is in an amount of 30 mL or more, and the polymerase chain reaction is performed while the reaction solution is stirred (hereinafter, also referred to as the "present method for amplifying a nucleic acid").

The thermal cycler of the present invention comprises: a temperature regulation unit which adheres to one or more reaction container(s) housing 30 mL or more of a reaction solution and regulates a temperature of the reaction solution, wherein the reaction container is made of a heat conductive material; and a stirring unit for stirring the reaction solution, wherein the temperature regulation unit comprises a heat source, a reaction container support plate which supports the reaction container by abutting an upper face of the reaction container, and a temperature sensor which measures an outside surface temperature of the reaction container or a temperature of the reaction solution (hereinafter, also referred to as the "present thermal cycler") for use in the present method for amplifying a nucleic acid.

### <PCR reaction>

The polymerase chain reaction described herein is a method of amplifying template DNA by performing 20 to 50 repetitive cycles as thermal cycles involving a series of steps consisting of thermal denaturation, annealing (primer binding), and DNA extension reaction in the presence of DNA polymerase, deoxyribonucleotide triphosphates (dNTPs), the template DNA, a forward primer, and a reverse primer. The polymerase chain reaction includes quantitative polymerase chain reaction (qPCR), reverse transcription-PCR (RT-PCR), single-base extension (SBE), multiplex single-base extension (MSBE), and ligase chain reaction-polymerase chain reaction (LCR-PCR).

Examples of the nucleic acid described herein can include DNA and RNA. The DNA may be single-stranded DNA or double-stranded DNA.

### (DNA polymerase)

The DNA polymerase described herein is preferably thermostable DNA polymerase. The thermostable DNA polymerase means thermophilic bacterium-derived DNA polymerase, and more than half of the DNA polymerase activity is maintained even if heat treatment is carried out, for example, at 70°C for 1 minute or longer. The DNA polymerase may be of type polI or of type α. Examples thereof can specifically include DNA polymerase of *Thermus aquaticus* (Taq), *Thermus thermophilus, Thermococcus litoralis, Thermococcus gorgonarius, Thermococcus kodakaraensis* KOD1, *Thermococcus cleftensis, Thermococcus eurythermalis, Thermococcus paralvinellae, Thermococcus sibiricus, Bacillus stearothermophilus, Methanothermus fervidus, Pyrococcus woesei, Pyrococcus furiosus* (PFU), *Pyrococcus* sp. GB-D, *Pyrococcus kukulkanii, Pyrococcus yayanosii, Pyrolobus fumarii, Aeropyrum pernix, Aquifex aeolicus, Sulfolobus tokodaii,* or *Methanopyrus kandleri.* These may be used as a mixture. Alternatively, DNA polymerase expressed in *E. coli* or the like in a genetic engineering manner may be used, or a commercially available product may be used. The DNA polymerase may be artificially synthesized in a genetic engineering manner or may be derived from an organism. The DNA polymerase used may be expressed in *E. coli* or the like by a gene recombination technique, then extracted, and if necessary, purified by ion chromatography or the like.

Examples of the concentration of the DNA polymerase in the reaction solution can include 2000 to 10000 U/pL, preferably 3000 to 8000 U/pL, when the ability to synthesize a strand from 10 ng of DNA (DNA obtained by replacing polynucleotide encoding EMrfp in PSRLaP-EMrfp described in the document of Nakamura et al. (Molecular Biotechnology 60, pages 912-923 (2018)) with a 711 bp polynucleotide encoding EGFP consisting of the nucleotide sequence represented by SEQ ID No: 1) per µL of DNA polymerase is defined as 1 U/µL.

### (Deoxyribonucleotide triphosphates (dNTPs))

The deoxyribonucleotide triphosphates (dNTPs) described herein can be a combination of dATP, dCTP, dGTP, and dTTP, and dUTP may be contained instead of dTTP. The concentrations of the dNTPs can be appropriately adjusted by the length of DNA to be amplified, the amount of DNA, and the type of a sequence. Each of the concentrations of dATP, dCTP, dGTP, and dTTP can be preferably 0.01 to 0.6 mM. The lower limit may be 0.02 mM, 0.05 mM, 0.1 mM, or 0.2 mM, and the upper limit may be 0.55 mM, 0.5 mM, or 0.45 mM.

### (Template DNA)

Examples of the template DNA described herein can include genomic DNA, cDNA, and plasmid DNA. Examples of the length of the template DNA can include 100 to 20,000 bp. The lower limit may be 200 bp, 300 bp, or 500 bp, and the upper limit may be 10,000 bp, 5,000 bp, or 3,000 bp. The template DNA may be present alone or may be incorporated in a vector such as a plasmid vector or a virus vector.

### (Primers)

The forward primer and the reverse primer described herein can each be an oligonucleotide that has complementarity enabling the primer to hybridize to the template DNA, and serves as a starting point of nucleic acid synthesis in PCR by annealing to a region to be amplified in the template DNA or an upstream sequence adjacent to the region.

Each of the lengths of the forward primer and the reverse primer can be appropriately adjusted by the length of DNA to be amplified, the amount of DNA, and the type of a sequence. Examples thereof can include 8 to 100 mer. The lower limit may be 12 mer, 15 mer, 20 mer, or 25 mer, and the upper limit may be 80 mer, 60 mer, 50 mer, 45 mer, or 40 mer.

The forward primer and the reverse primer may be modified. These primers may each be labeled with, for example, avidin or a fluorescent material, or may be a phosphorothioated or phosphoramidited oligonucleotide derivative.

### (Buffer solution)

The buffer solution described herein is not particularly limited as long as the buffer solution can be used in PCR reaction. Examples thereof can include a Tris hydrochloride buffer solution, a Tris acetate buffer solution, a bis tris buffer solution, a HEPES buffer solution, and a MOPS buffer solution. If necessary, the pH of the buffer solution may be adjusted with hydrochloric acid or the like, or EDTA or a metal ion such as Mg²⁺ or K⁺ may be added to the buffer solution. The concentration of the buffer solution may be appropriately adjusted by the length of DNA to be amplified, the number of thermal cycles, etc. Examples thereof can preferably include 0.02 M to 1.5 M. The lower limit may be 0.05 M, 0.1 M, 0.15 M, 0.17 M, 0.2 M, 0.3 M, 0.4 M, 0.5 M, 0.6 M, or 0.7 M, and the upper limit may be 1.2 M, 1 M, 0.9 M, or 0.8 M. Examples of the concentration of the buffer solution can include 0.4 to 1.5 M. The concentration of buffer solution in this specification means the concentration (M) of the component in the buffer solution that exhibits buffering action. Specifically, in the case of Tris hydrochloric acid buffer, it means the concentration of Tris hydrochloric acid.

The pH of the buffer solution is preferably 7.0 to 10.0. The lower limit may be 7.5, 8.0, 8.5, or 9.0, and the upper limit may be 9.5 or 9.3. Examples of the pH of the buffer solution can include 8.5 to 10.0, preferably 9.0 to 9.3.

### (Reaction solution)

The amount of reaction solution described herein are not particularly limited to, but it can be 30 mL or more. The lower limit may be 50 mL, 100 mL, 150 mL, 180 mL, 200 mL, 220 mL, 250 mL, or 300 mL, and the upper limit of the reaction solution may be 10 L, 5 L, 4 L, 3 L, or 2 L. In this context, the amount of the reaction solution is the total amount of a reaction solution in which nucleic acid amplification reaction is performed by PCR at the same thermal cycles. That is, the reaction solution may be housed in one reaction container, or may be housed in divided portions in two or more reaction containers as long as the respective thermal cycles of the reaction containers can be controlled under almost the same temperature conditions. For example, one reaction container housing 30 mL or more of a reaction solution may be placed on a heat conductive plate in contact with a heat source so as to control the temperature of the reaction container, or three reaction containers each housing 10 mL of a reaction solution may be provided, and these three reaction containers can be placed on a heat conductive plate so that the bottom surfaces of the three reaction containers are adhered to the heat conduction plate, and so as to control the respective inside temperatures of the reaction containers under the same conditions. The reaction solution may further contain dimethyl sulfoxide, bovine serum albumin, glycerol, heparin, trehalose, a nonionic surfactant, ammonium sulfate, betaine, etc., without inhibiting nucleic acid amplification by PCR. The reaction solution may contain a probe in order to detect an amplification product. Examples of the probe can include a TaqMan probe and a cycling probe.

The reaction solution may further contain a label such as a dye binding to a phosphate group on a DNA surface or a dye intercalating between bases, in order to detect or monitor an amplification product. Examples of the dye binding to a phosphate group on a DNA surface or a dye intercalating between bases can include ethidium bromide, a cyanine dye, SYBR(R) Green, fluorescent coumarin, ruthenium, and ethidium bromide.

### (Polymerase chain reaction)

The polymerase chain reaction may have two-step cycles that involve thermal denaturation followed by annealing and extension reaction performed at the same temperature, or three-step cycles that involve thermal denaturation followed by annealing and extension reaction performed at different temperatures.

Examples of the temperature of the thermal denaturation in the polymerase chain reaction described herein can include 84 to 98°C. The lower limit may be 86°C, 87°C, 88°C, 89°C, 90°C, 91°C, 92°C, 93°C or 94°C, and the upper limit may be 97°C, 96°C, or 95°C. Examples of the time of the thermal denaturation can include 1 to 90 seconds. The lower limit may be 5 seconds, 7 seconds, or 10 seconds, and the upper limit may be 60 seconds or 30 seconds.

Examples of the temperature of the annealing in the polymerase chain reaction described herein can include 50 to 75°C. The lower limit may be 55°C, 57°C, or 60°C, and the upper limit may be 72°C, 70°C, or 68°C. Examples of the time of the annealing can include 10 to 60 seconds. The lower limit may be 15 seconds, 20 seconds, or 25 seconds, and the upper limit may be 50 seconds, 45 seconds, or 40 seconds.

Examples of the temperature of the extension reaction in the polymerase chain reaction described herein can include 50 to 80°C. The lower limit may be 55°C, 60°C, or 62°C, and the upper limit may be 75°C, 70°C, or 68°C. Examples of the time of the extension reaction can include 10 seconds to 20 minutes. The lower limit may be 30 seconds, 1 minute, or 2 minutes, and the upper limit may be 15 minutes, 10 minutes, 5 minutes, or 3 minutes.

In the case of performing the polymerase chain reaction described herein by two-step cycles, examples of the temperature of the annealing and the extension reaction can include 55 to 80°C. The lower limit may be 55°C, 60°C, or 62°C, and the upper limit may be 72°C, 70°C, or 68°C. Examples of the time of the annealing and the extension reaction can include 20 seconds to 20 minutes. The lower limit may be 30 seconds, 1 minute, or 2 minutes, and the upper limit may be 15 minutes, 10 minutes, 5 minutes, or 3 minutes.

Examples of the thermal cycles of thermal denaturation, annealing, and extension reaction can include 20 to 50 cycles. The lower limit may be 22 cycles or 24 cycles, and the upper limit may be 40 cycles, 38 cycles or 36 cycles. Examples of the time of one cycle, i.e., the respective times of the steps in one cycle of thermal denaturation, annealing, and extension reaction and a temperature regulation time, can include 3 minutes to 30 minutes. The lower limit may be 5 minutes, 7 minutes, or 10 minutes, and the upper limit may be 25 minutes, 20 minutes, or 15 minutes.

In the method for amplifying a nucleic acid according to the present invention, a typical example of two steps of the polymerase chain reaction is as follows.

| | |
|---|---|
| (1) initial thermal denaturation ↓ | 90°C for 10 seconds |
| (2) thermal denaturation | 90 or 92°C for 10 or 30 seconds |
| (3) annealing and extension reaction | 60 or 65°C for 2.5 minutes |
| (4) 30 cycles of (2) and (3) | |

In the method for amplifying a nucleic acid according to the present invention, a typical example of three steps of the polymerase chain reaction is as follows.

| | |
|---|---|
| (1) initial thermal denaturation ↓ | 90°C for 10 seconds |
| (2) thermal denaturation | 90 or 92°C for 10 or 30 seconds |
| (3) annealing | 65°C for 30 seconds |
| (4) extension reaction | 72°C for 2.5 minutes |
| (5) 30 cycles of (2) to (4) | |

### (Reaction container)

The reaction container can house the reaction solution. Its shape can be an arbitrary shape such as a sac-like, box-like, cylindrical, or flat plate-like shape. A sac-like container having a planar shape is preferred from the viewpoint of heat conduction efficiency. The reaction container may be sealable or partially open to the reaction liquid. Furthermore, the reaction container is provided with an opening into which the reaction solution is added, and after containing the reaction liquid, the opening may be closed after housing of the reaction solution so as not to leak the reaction solution.The opening can be closed after housing of the reaction solution so as not to leak the reaction solution. Examples of the closing method can include heat seal and adhesion with an adhesive.

The material for the reaction container can have heat conductance that allows the heat of a heat source to be conducted to the reaction solution via the reaction container. Examples thereof can include a polyolefin resin such as polyethylene and polypropylene, a polyester resin such as polyethylene terephthalate, a styrene resin such as polystyrene, and a metal such as aluminum, copper, silver, and gold. Examples of the thickness of the reaction container can include 0.01 to 1.5 mm and 50 µm to 3 cm for a metal in the case of a resin such as a polyolefin resin, a polyester resin, and a styrene resin. In the case of a resin, plural types of resins may be layered. Examples of the metal can include a metal having a heat conductivity of 50 W/m•K or more. In the case of using two or more reaction containers at the same time, it is preferred to use reaction containers of the same material from the viewpoint of uniformly controlling reaction solutions.

The reaction container preferably has the heat conductance as well as stretchability. The stretchability thus possessed can improve the close attachment between the reaction container and a heat source or a heat conductive plate adhering to the heat source, and enhance heat conduction efficiency. Also, the stretchability thus possessed enables the reaction container to be swollen when water vapor is generated at the thermal denaturation stage, and the swollen reaction container to be shrunk at the anneal stage or the extension reaction stage. Since the temperature may reach 95°C or higher at the thermal denaturation stage, the reaction container is preferably made of a material resistant to a high temperature of 95°C, preferably 100°C, i.e., a material free from reaction solution leakage ascribable to the breakage of the reaction container by a high temperature.

The reaction container may be provided with a temperature sensor holding part for holding a temperature sensor and facilitating measuring with the temperature sensor the outside surface temperature of the reaction container or the temperature of the reaction solution. Examples of the temperature sensor holding part can include a temperature sensor holding part that is formed in a pocket form on the outside surface of the reaction container so as to house a thermometry part within the pocket and encompass the resultant in the reaction container.

### (Stirring of reaction solution)

Examples of the method for stirring the reaction liquid as described herein can include, but are not particularly limited to, a method of oscillating the reaction container itself, a method of providing a stirring machine such as a propeller stirring machine or a jet stirring machine in the reaction container, and a method of irradiating the reaction solution with ultrasonic wave.

Examples of the method for oscillating the reaction container can include a method using a shaker. Examples thereof can include a method of directly or indirectly fixing or installing the reaction container on the upper side of a shaker so as to convey a shake motion generated by the shaker to the reaction container. Examples of the method for fixing or installing the reaction container can include, but are not particularly limited to, a method of fixing or installing the reaction container onto a shake plate of the shaker, or a shake shaft fixed to the shake plate, to which a shake motion is conveyed. The reaction container may be oscillated by a rotational motion or a reciprocal motion in the shaker. The direction of the motion may be a horizontal direction or a perpendicular direction, or a combination thereof. In the case of a rotational motion, examples of the rotating speed can include 10 to 200 rpm. The rotating speed may be 50 to 100 rpm. An additional unit may be disposed between the reaction container and the shake plate without impairing the shake of the reaction container. For example, a heat source or a heat conductive plate mentioned later may be disposed between the reaction container and the shake plate.

In the method of providing a stirring machine such as a propeller stirring machine or a jet stirring machine in the reaction container, examples of the stirring machine can include a propeller stirring machine and a jet stirring machine. In this case, the propeller stirring machine or the jet stirring machine can be positioned within the reaction container with the reaction container hermetically sealed such that the reaction solution does not leak out of the reaction container.

Other examples of the method for stirring the reaction solution can include a method of putting a magnetic stirring bar in the reaction container and stirring through the use of magnetic force from the outside of the reaction container using a magnetic stirrer. The magnetic stirring bar can have magnetism. The magnetic stirring bar may have a rod-like or elliptic shape and may be covered with a fluorine resin or the like. The magnetic stirring bar is capable of rotating or sliding through the magnetic force from the outside of the reaction container to stir the reaction solution.

In agitating the reaction solution by oscillating the reaction container, it is preferable to include a predetermined amount or more of the reaction solution in the reaction container in order to improve the agitation efficiency. Although the amount of reaction solution can be adjusted according to the bottom area of the reaction container, the height of the reaction solution in the reaction container, i.e., the vertical thickness, may be 2 mm or more, preferably 3 mm, 4 mm, or 5 mm or more when the reaction container is standing still.

In the case of using a hermetically sealed container, 1 to 90% of a gas is preferably contained in the reaction container at a stage before the start of PCR reaction. The lower limit may be 3%, 5%, 7%, or 10%, and the upper limit may be 80%, 70%, 60%, 50%, 40%, 30, or 20%. In this context, the content of the gas in the reaction container is a content in a state where the reaction container is closed before PCR reaction is performed and after the reaction solution is added into the reaction container. In consideration of heat transfer efficiency, it may be preferred that the reaction container should be hermetically sealed with the reaction solution without containing a gas. However, if the reaction container is filled with a reaction solution with a large volume, stirring efficiency is poor even if the reaction solution is stirred, for example, by oscillating the reaction container itself. Accordingly, when a predetermined amount of a gas is contained in the reaction container, the gas moves quickly in the reaction container by oscillating the reaction container. As a result, the stirring efficiency of the reaction solution is improved. The gas thus contained facilitates suppressing the leakage of the reaction solution in taking the reaction solution out of the reaction container after PCR reaction. The gas is not particularly limited as long as the gas does not inhibit PCR reaction. Examples thereof can include air, carbon dioxide, and nitrogen. Instead of directly containing gas, a bag housing a gas and/or a liquid may be contained in the reaction container. A stirring auxiliary material, such as beads, which moves freely in the reaction solution may be contained in the reaction container in order to improve the stirring efficiency of the reaction solution.

### (Temperature control of reaction container)

Examples of the method for controlling the temperature of the reaction container can include a method using a thermal cycler comprising a temperature control unit capable of controlling the temperature of the reaction container by abutting at least a portion of the reaction container. It is preferred to use the thermal cycler of the present invention. Hereinafter, a description will be given of the temperature control of the reaction container in the method for amplifying a nucleic acid according of the present invention, and a main configuration of the temperature control unit in the thermal cycler of the present invention.

The temperature regulation unit comprises a heat source, a reaction container support plate which supports the reaction container by abutting the upper face of the reaction container, and a reaction container temperature sensor which measures the outside surface temperature of the reaction container or the temperature of the reaction solution. Examples of the control of the temperature of the reaction container can include a method of heating or cooling the reaction solution by allowing the reaction container to abut the heat source directly or indirectly via a heat conductive plate. The heat source may be allowed to abut any one or more of the underside, the lateral face, and the upper face of the reaction container and may be allowed to abut only the underside, both the underside and the upper face, or the underside and the whole or a portion of the lateral face. In the case of allowing the heat source to abut the upper face, the reaction container may be symmetrically provided with upper and lower heat sources, for example, as described in patent document 4 described above. Since a gas may accumulate on the upper side in the reaction container due to water vapor generated in the PCR reaction solution at the thermal denaturation stage in PCR reaction, the heat source preferably abuts at least the underside of the reaction container, from the viewpoint of the temperature control or heat conduction efficiency of the PCR reaction solution, and may abut only the underside.

The upper face of the reaction container, i.e., a symmetrical face of the underside of the reaction container, is preferably provided with a reaction container support plate for supporting the reaction container so that the reaction container is supported thereby. Examples of the material for the reaction container support plate can include, but are not particularly limited to, a metal such as aluminum and copper, and a plastic. Alternatively, two heat sources may be provided. One of the heat sources can serve as a reaction container support plate, and the reaction container may be sandwiched between the heat sources on the upper face and the underside.

The reaction container support plate may be made of a heat insulation material or may be a heater such as an electrothermal heater. In the case of a heater, easy temperature control of thermal denaturation, anneal, and extension reaction as well as reduction in the time of thermal cycles is accomplished, for example, by controlling the temperature to near the temperature of the anneal stage.

The heat source is not particularly limited as long as the heat source contained is capable of absorbing and applying heat. Examples of such a heat source can include a Peltier element and a heater such as an electrothermal heater. The number of heat sources may be appropriately adjusted according to the amount of the PCR reaction solution, and one or more, for example, two or more, three or more, five or more, eight or more, 10 or more, or 20 or more heat sources may be provided. In other words, one reaction container may be provided, only on the underside, with a heat source. When the reaction container has a wide area, a plurality of heat sources may be apposed in the horizontal direction so as to absorb heat from and apply heat to the outside surface of the reaction container in a shorter time.

The heat source such as a Peltier element or a heater may come into direct contact with the reaction container or may have a heat conductive plate on a face in contact with the reaction container. Heat released from the heat source via the heat conductive plate is conveyed to the reaction solution via the reaction container. It is also preferred that the Peltier element and the heat conductive plate should adhere to each other with a heat conductive filler such as heat conductive grease, from the viewpoint of heat conductance.

Examples of the material for the heat conductive plate can include a material having high heat conductance, for example, a metal such as aluminum, copper, silver, and gold. The heat conductive plate preferably has a plate-like shape, and may have a projection, a depression, an asperity, a fan shape, or a slope on a face that adheres to the reaction container because the stirring efficiency of the reaction container or an adhesion area for the reaction container is improved. The thickness of the heat conductive plate can be appropriately adjusted depending on the material. Examples thereof can include 0.1 mm to 5 cm. The thickness may be 0.5 mm to 3 cm or 1 mm to 1 cm. The length and breadth of the heat conductive plate can be appropriately regulated according to the length and breadth of the reaction container and the number of heat sources. One heat source may have one heat conductive plate, or a plurality of heat sources may have one heat conductive plate. Examples of the shape of the heat conductive plate can include a square, a rectangle, and an ellipse. It is preferable for the temperature control that the heat conductive plate abuts on 50% or more of the bottom surface of the reaction container, and may be 60% or more, 70% or more, 80% or more, 90% or more, 95% or more of the bottom area. In addition, since the reaction solution is oscillated, the area where the heat conductive plate is not abut on the reaction solution through the reaction container may be 5% or more, 10% or more, 15% or more, 20% or more, 30% or more of the bottom surface of the reaction vessel.

The heat conductive plate preferably has a depression or a through-hole so as not to directly convey the heat of the heat source thereto when a thermometry part of a temperature sensor which measures the outside surface temperature of the reaction container is disposed to the heat conductive plate. The shape of the depression or the through-hole is not particularly limited and is, for example, a circle, a square, a rectangle, an ellipse, or a conical shape. The size (minor diameter in the case of a rectangle or an ellipse) of the depression or the through-hole may be preferably a size by which the thermometry part of the temperature sensor does not come into contact with the heat conductive plate. For example, the size of the depression or the through-hole may be preferably large enough to keep the shortest distance between the thermometry part of the temperature sensor and the heat conductive plate 0.5 mm or larger, 1 mm or larger, 2 mm or larger, 3 mm or larger, 4 mm or larger, 5 mm or larger, 7 mm or larger, 10 mm or larger, or 15 mm or larger. In other words, the radius or minor axis of the depression or through-hole may be 0.5 mm or more, 1 mm or more, 2 mm or more, 3 mm or more, 4 mm or more, 5 mm or more, 7 mm or more, 10 mm or more, or 15 mm or more. The position of the depression or the through-hole may be any position in the heat conductive plate and is preferably at or around the center. Instead of providing the depression or the through-hole, a heat insulation sheet such as a sheet made of silica aerogel as a material may be affixed to the heat conductive plate, and the thermometry part of the temperature sensor can be disposed on the heat insulation sheet, or the thermometry part of the temperature sensor may be affixed to the reaction container using the heat insulation sheet.

Furthermore, the heat conductive plate may be provided with a hollow part to improve the efficiency of heat radiation from the reaction container. The area of such a hollow part is not restricted, but may be from 1 cm² to 9 cm², with a lower limit of 1.5 cm² or 2 cm² and an upper limit of 8 cm², 5 cm^{2,} or 4 cm². The hollow part may have a reticular, lattice, or linear hollow part as necessary to prevent the reaction container from sinking.

The Peltier element may have a heat sink, a fin, a fan, and/or a water cooling unit on a face opposite to the face in contact with the reaction container. It is preferred that the Peltier element and the heat sink should be closely attached to each other with a heat conductive grease.

A power control part, an operation part, a current control part having a power supply part, and a temperature sensor are connected to the temperature regulation unit.

Examples of the temperature sensor can include a reaction container temperature sensor which measures the outside surface temperature of the reaction container, a reaction solution temperature sensor having a thermometry part positioned within the reaction container, wherein the thermometry part comes into direct contact with the reaction solution and measures the temperature of the reaction solution, and a heat conductive plate temperature sensor which measures the heat source or temperature of the heat conductive plate abutting the reaction container. Any one of these temperature sensors may be used, or any combination thereof may be used. It is preferred to have at least a reaction container temperature sensor or a reaction solution temperature sensor.

Examples of the reaction container temperature sensor which measures the outside surface temperature of the reaction container can include a low-profile temperature sensor affixable to the outside surface of the reaction container, a probe-type temperature sensor having a thermometry part on its tip, and a radiation temperature sensor such as an infrared temperature sensor capable of measuring the outside surface of the reaction container. As the probe-type temperature sensor, a temperature sensor that can be expanded and contracted by a spring or the like may be used. When the heat conductive plate is provided with a through hole, the temperature detection part of the probe-type temperature sensor is arranged in the through hole, and when the reaction container is placed on the heat conductive plate, the outer surface of the reaction container can be measured by bringing the temperature detection part into contact. In the case of maintaining a sterile state of the reaction solution, such as the case of using a sterilized reaction solution, it is preferred to use a reaction container temperature sensor or a heat conductive plate temperature sensor.

Examples of the reaction solution temperature sensor having a thermometry part positioned within the reaction container, wherein the thermometry part comes into direct contact with the reaction solution and measures the temperature of the reaction solution can include a wireless temperature sensor, and a rod-like temperature sensor that can be disposed such that the thermometry part is positioned within the liquid of the reaction container through the reaction container. In the case of using a wireless temperature sensor, a method can be employed in which the wireless temperature sensor can be put into the reaction solution either directly or after being subjected to water proof and/or heat resistance treatment.

The number of the temperature sensor may be one or more. In the case of using the reaction container temperature sensor, the position where the temperature sensor abuts the reaction container can be a position that falls within the outside surface of the reaction container and comes into contact with the reaction solution via the reaction container, and is preferably the underside and/or the lateral face. In addition, in the case of the underside, the position is not particularly limited whether it is the central portion or the peripheral portion. Temperature control for conventional PCR is mainly performed based on a temperature measured by a heat conductive plate temperature sensor. This is probably because, since a conventional reaction solution is on the order of 10 to 50 µL, the heat of the heat source or heat conductive plate abutting the reaction container is easily conducted to the whole reaction solution. However, when the reaction solution is in an amount of 30 mL or more, the temperature of the reaction solution differs easily from the temperature of the heat source or heat conductive plate. Hence, for facilitating controlling the temperature of the reaction solution, it is preferred to measure the outside surface temperature of the reaction container or the temperature of the reaction solution.

Information on the temperature detected by the temperature sensor is sent to the current control part. Then, current to be supplied to the heat source such as a Peltier element from the power control part is determined in the operation part. Specifically, the current control part has a function as a power supply for the heat source such as a Peltier element and also controls the temperature of the heat source such as a Peltier element or the heat conductive plate by regulating the current to be supplied to the heat source such as a Peltier element based on data on the temperature of the outside surface of the reaction container and/or the heat conductive plate detected by the temperature sensor. For suppressing overshoot or undershoot in temperature control, it is preferred to perform the control by PID control or the like.

In the operation part, for example, thermal cycle conditions such as the each of temperatures and times of thermal denaturation, annealing, and extension reaction, and the number of thermal cycles are preset, and current is controlled so as to perform heating when the temperature of the outside surface of the reaction container or the reaction solution is lower than a set temperature and so as to perform cooling when this temperature is higher than the set temperature. In this context, the temperature of the reaction solution varies easily in large-volume PCR. A nucleic acid cannot be specifically amplified unless the reaction temperature and reaction time of each step in thermal cycles shown in Figure 1 are accurately controlled. At the thermal denaturation stage, thermal denaturation of the template DNA fails at a temperature lower than the set temperature while a component, such as DNA polymerase, contained in the reaction solution is denatured at a temperature higher than the set temperature for a long time. In the annealing, nonspecific binding is increased at a temperature lower than the set temperature while primers dissociate from template DNA at a temperature higher than the set temperature. In the extension reaction, the function of DNA polymerase is reduced at a temperature lower or higher than the set temperature. As for the reaction time, a longer time per thermal cycle is more likely to deactivate DNA polymerase. Hence, the way to accurately control the reaction solution at the set temperature in a short time is very important.

When the temperature regulation unit has a reaction container temperature sensor and a heat conductive plate temperature sensor, it is preferred to control the outside surface temperature of the reaction container measured by the heat conductive plate temperature sensor so as not to be much higher or lower than the set temperature, i.e., so as not to cause overshoot or undershoot in increase or decrease in temperature, while temperature control such that the temperature of a heat source or a heat conductive plate measured by the reaction container temperature sensor satisfies each condition for thermal cycles. Since a conventional thermal cycler commonly performs nucleic acid amplification in 50 µL or less of a reaction solution where heat conduction rapidly takes place, the temperature management of a heat source or a reaction container is widely practiced by measuring the temperature of a heat source or a reaction container in contact with a reaction container, not the reaction container. However, when the reaction solution has a large volume, the temperature of the heat source or reaction container and the outside surface temperature of the reaction container differ easily. Hence, use of these two sensors enables control of a temperature more approximated to the temperature of the reaction solution.

In transition from thermal denaturation to annealing reaction, transition from annealing to extension reaction, and/or transition from extension reaction to thermal denaturation in polymerase chain reaction, increase or decrease in the temperature of the outside surface of the reaction container or the reaction solution can be controlled at 0.05°C/sec or more. This temperature increase or temperature decrease can be adjusted according to the size of the reaction container, the amount of reaction solution, and other factors. The temperature increase is preferably performed at 0.1 to 6°C/sec. The lower limit may be 0.08, 0.1, 0.2, 0.3, 0.4 or 0.5°C/sec, and the upper limit may be 5, 4, 3, 2, 1.5, 1.2, 1, 0.8, 0.5, 0.2 or 0.1°C/sec, though the temperature increase or decrease is not particularly limited thereto. This temperature increase or temperature decrease can be adjusted according to the size of the reaction container, the amount of reaction solution, and other factors. The temperature decrease is preferably performed at 0.1 to 6°C/sec. The lower limit may be 0.2, 0.3, 0.4 or 0.5°C/sec, and the upper limit may be 5, 4, 3, 2, 1.5, 1.2, 1, 0.8, or 0.7°C/sec, though the temperature increase or decrease is not particularly limited thereto. Moreover, it is preferable that the speed of temperature decrease is faster than the speed of temperature increase. Specifically, it may be 1.2, 1.5, 1.8 or 2 times faster than the speed of temperature increase.

A plurality of temperature regulation units may be provided on a set temperature basis in PCR thermal cycles. For example, the temperature regulation unit may be provided for each step of thermal denaturation, anneal, and extension reaction, and temperature control can be performed by the temperature regulation unit for each step. Specifically, in the case of performing PCR reaction by 3 steps, four temperature regulation units can be provided: temperature regulation unit A for thermal denaturation, temperature regulation unit B for anneal, temperature regulation unit C for extension reaction, and preheating or cooling temperature regulation unit D for performing temperature increase or decrease in a shorter time such that the reaction container attains the set temperature of the next step in a short time. A method of controlling the temperature by the following cycles can be adopted.

1. Thermal denaturation for a predetermined time in the temperature regulation unit A for thermal denaturation adjusted to the set temperature of thermal denaturation.
2. Transfer of the reaction container to the preheating or cooling temperature regulation unit D adjusted in advance to the anneal temperature or lower, for example, 0°C.
3. Transfer of the reaction container to the temperature regulation unit B for anneal adjusted to the anneal temperature after the temperature of the reaction container approaches the set temperature of the next step, i.e., anneal.
4. Anneal for a predetermined time in the temperature regulation unit B for anneal.
5. Transfer of the reaction container to the preheating or cooling temperature regulation unit D adjusted to the set temperature or higher of extension reaction, for example, 90°C.
6. Transfer of the reaction container to the temperature regulation unit C for extension reaction adjusted to the extension reaction temperature after the temperature of the reaction container approaches the set temperature of the next step, i.e., extension reaction.
7. Extension reaction for a predetermined time in the temperature regulation unit C for extension reaction.
8. Transfer of the reaction container to the preheating or cooling temperature regulation unit D adjusted to the set temperature or higher of thermal denaturation, for example, 98°C.
9. Transfer of the reaction container to the temperature regulation unit A for thermal denaturation adjusted to the thermal denaturation temperature after the temperature of the reaction container approaches the temperature of the next step, i.e., thermal denaturation.

Accurate temperature control in a shorter time as well as PCR reaction is accomplished by performing one cycle from the above steps 1 to 9 predetermined times. The temperature control of the reaction container may omit the above step 8 and proceed to step 9' instead of step 9 "transfer of the reaction container to the temperature regulation unit A for thermal denaturation adjusted to the thermal denaturation temperature" because heating is performed in a short time as compared with cooling. The temperature control may omit the above steps 5 and proceed to step 6' instead of step 6 "Transfer of the reaction container to the temperature regulation unit C for extension reaction adjusted to the extension reaction temperature" because the set temperatures of anneal and extension reaction are similar temperatures.

Temperature increase or decrease between the steps can be handled in a shorter time through the use of the preheating or cooling temperature regulation unit. Therefore, the total reaction time of the PCR reaction can be shortened. In addition, reduction in the function of a component, such as DNA polymerase, contained in the reaction solution can be suppressed. The reaction solution may be stirred by use of a method of providing a support table that supports all the temperature regulation units and rotating or reciprocating the support table by the power of a shaker or a motor, etc., or by use of a stirring machine, a stirrer, shaking mechanism or an ultrasonic wave, etc. for each temperature regulation unit.

Likewise, in the case of performing PCR reaction by two steps, three temperature regulation units can be provided: temperature regulation unit A for thermal denaturation, temperature regulation unit B for anneal and extension reaction, and preheating or cooling temperature regulation unit D. A method of controlling the temperature by the following cycles can be adopted.
1. Thermal denaturation for a predetermined time in the temperature regulation unit A for thermal denaturation adjusted to the set temperature of thermal denaturation.
2. Transfer of the reaction container to the preheating or cooling temperature regulation unit D adjusted in advance to the anneal and extension reaction temperature or lower, for example, 0°C.
3. Transfer of the reaction container to the temperature regulation unit B for anneal and extension reaction adjusted to the anneal and extension reaction temperature after the temperature of the reaction container approaches the set temperature of the next step, i.e., anneal and extension reaction.
4. Anneal and extension reaction for a predetermined time in the temperature regulation unit B for anneal and extension reaction.
5. Transfer of the reaction container to the preheating or cooling temperature regulation unit D adjusted to the set temperature or higher of thermal denaturation, for example, 98°C.
6. Transfer of the reaction container to the temperature regulation unit A for thermal denaturation adjusted to the thermal denaturation temperature after the temperature of the reaction container approaches the temperature of the next step, i.e., thermal denaturation.

Accurate temperature control in a shorter time as well as PCR reaction is accomplished by performing one cycle from the above steps 1 to 6 predetermined times. The temperature control of the reaction container may omit the above step 5 and proceed to step 6' instead of step 6 "transfer of the reaction container to the temperature regulation unit A for thermal denaturation adjusted to the thermal denaturation temperature" because heating is performed in a short time as compared with cooling.

### (Thermal cycler)

For the present thermal cycler, a temperature regulation unit is as mentioned above. The aspects of the temperature regulation unit will be described in more detail. The temperature regulation unit of reaction container comprises a heat source, a reaction container support plate which supports the reaction container by abutting the upper face of the reaction container, and a reaction container temperature sensor which measures the outside surface temperature of the reaction container or the temperature of the reaction solution. In a typical example, a plurality of heat sources are disposed in parallel in the horizontal direction, and a heat conductive plate on which the reaction container is placed adheres to the upper side of the heat sources via heat conductive grease. Given space to house the reaction container is provided on the upper side of the heat conductive plate. The reaction container support plate is disposed on the upper side of this space. An anti-displacement plate for preventing the displacement of the reaction container by the oscillation of the reaction container may be installed in the vertical direction at the end of the heat conductive plate.

The reaction container support plate is horizontally provided on the upper side of the reaction container. Its distance from the heat source or the heat conductive plate adhering to the heat source may be appropriately adjusted according to the size of the reaction container used or the volume of the reaction container. The reaction container support plate may have a structure that supports as abutting the reaction container under its own weight, or may be secured or maintained in a position abutting the reaction container using a screw jack, wire or the like. A shaft that supports the reaction container support plate may be provided, and the shaft may be secured or maintained by a cylinder such as an air cylinder or a robo-cylinder, an extendable mechanism such as a spring. In addition, the reaction container support plate may be positioned to allow a predetermined pressure to be applied to the reaction container to improve adhesion between the reaction container and the heat source or heat conductive plate.

In addition, the reaction container support plate can be provided with securing or maintaining means at a position such that a predetermined gap is provided between the reaction container support plate and the reaction container, for example, a gap of 1 cm or more, 2 cm or more, 3 cm or more, 4 cm or more, 5 cm or more, 7 cm or more, or 10 cm or more. With such a configuration, a gap may be provided between the reaction container support plate and the reaction container, for example, during the temperature lowering step in the thermal cycle, ie, the step between after thermal denaturation and annealing. By providing such a gap in the temperature lowering step, the upper surface of the reaction container is exposed to the air, which makes it possible to efficiently radiate heat from the reaction container and reduces the time per thermal cycle. The means for securing or maintaining the reaction container support plate above the reaction container with a predetermined gap is not particularly limited, but a method using a screw jack or a wire capable of electric automatic lifting control according to the thermal cycle, a method using a cylinder such as an air cylinder or ROBO cylinder, or a method using a cam mechanism can be mentioned. Alternatively, when supporting the reaction container support plate by its own weight by using a shaft which support the reaction container support plate or using a wire, a stopper such as a set collar can be provided on the shaft, and the position of the stopper can be adjusted so that when the reaction container is shrunk during cooling, a gap is formed between the upper surface of the reaction container and the reaction container support plate.

The heat source and the heat conductive plate are preferably connected through a screw or the like from the viewpoint of heat conduction efficiency for the reaction container. The heat source is supported by a heat source support shaft. When the heat conductive plate and the heat source are connected, only the heat conductive plate may be supported by the heat source support shaft. The reaction container support plate on the upper side may further be supported by this heat source support shaft.

Next, examples of the stirring unit can include a unit capable of oscillating the reaction container. For example, a shaker can be used. The reaction container can be oscillated by fixing the temperature regulation unit to the shaker so as to convey the shake of the shaker to the reaction container. Examples of the shaker can include a shaker capable of rotary shake or reciprocal shake. In the case of a rotary shake, the rotating speed is as described in the above section "Stirring of reaction solution."

In the case of putting a stirring bar into the reaction container as described above, a stirrer can be used as the stirring unit. In this case, the temperature regulation unit is provided with space that does not hinder magnetism between the stirrer and the stirring bar in the reaction container. Specifically, in the case of installing a plurality of heat sources in parallel in the horizontal direction, space is provided between the heat sources, and the stirring bar is actuated by the stirrer via the space.

An ultrasonic apparatus may be used as the stirring unit. The reaction solution in the reaction container is stirred by irradiating the reaction container with ultrasonic wave. In this case, space that does not hinder ultrasonic wave is provided between the ultrasonic apparatus and at least a portion of the reaction container. Specifically, in the case of installing a plurality of heat sources in parallel in the horizontal direction, space is provided between the heat sources, and the reaction container is irradiated with ultrasonic wave by the ultrasonic apparatus via the space.

The connection between the temperature regulation unit and the stirring unit is not particularly limited. In the case of using, for example, a shaker, as the stirring unit, a shake shaft is provided in the vertical direction on a shake table, and the temperature regulation unit can be fixed to the shake shaft. In the case of using a Peltier element as the heat source and providing a cooling fan, the cooling fan and the shake table can be spaced with a predetermined width, preferably 3 cm or more, so as not to closely attach the cooling fan to the shake table.

### Examples

Hereinafter, the present invention will be described more specifically with reference to Examples. However, the technical scope of the present invention is not limited by these examples.

### [Example 1] Preparation of DNA polymerase Pfu

In Examples below, the DNA polymerase used was DNA polymerase Pfu expressed in *E*. *coli,* and commercially available PrimeSTAR(R) GXL (Takara Bio Inc.) and KOD Fxneo (Toyobo Co., Ltd.). The DNA polymerase Pfu expressed in *E*. *coli* was prepared by the following method.

First, *E. coli* HB101 competent cells (Takara Bio Inc.) were mixed with a plasmid containing a nucleotide sequence (SEQ ID No: 2) encoding DNA polymerase Pfu, and placed in an ice bath for 10 minutes and at 40°C for 5 seconds. Then, 50 µL of sterile water was added thereto, and the cells were cultured at 37°C for 24 hours in a plate culture medium containing yeast extracts, peptone, and ampicillin to obtain a transformant colony. This colony was added to a culture medium containing yeast extracts, peptone, and ampicillin and cultured at 200 rpm for 24 hours. After the culture, 500 µL of the culture medium was collected into a tube and centrifuged, and the supernatant was discarded. Then, a buffer solution containing 1% Triton X-100 was added thereto, followed by incubation. Then, the mixture was further centrifuged to obtain a supernatant as a DNA polymerase Pfu solution (hereinafter, also simply referred to as a "Pfu solution"). In the case of performing SDS-PAGE, a supernatant was obtained by centrifugation, mixed with a 6× sample buffer, and left standing at 97°C for 5 minutes. Results of analyzing 50 µL of the DNA polymerase Pfu solution by SDS-PAGE are shown in Figure 2. As is evident from Figure 2, a protein of 90 kDa, which is the molecular weight of the Pfu protein, was obtained.

### [Example 2] Study on amount of reaction solution and time

The amount of a reaction solution for DNA amplification efficiency, and a reaction time for DNA amplification efficiency were examined. PCR was performed using the Pfu solution prepared in Example 1, and commercially available PrimeSTAR(R) GXL (Takara Bio Inc.) and KOD Fxneo (Toyobo Co., Ltd.) as DNA polymerase.

To a 200 µL microtube, 10, 25, 50, 75, 100, 125, 150, or 200 µL of a reaction solution was added, and PCR was performed. The template DNA used was a plasmid containing 711 bp DNA encoding EGFP and consisting of the nucleotide sequence represented by SEQ ID No: 1. The primers used were a forward primer consisting of the nucleotide sequence represented by SEQ ID No: 3 and a reverse primer consisting of the nucleotide sequence represented by SEQ ID No: 4, which were designed so as to amplify the 711 bp DNA encoding EGFP. The composition (final concentration) of the PCR reaction solution is given below.

### <PCR reaction solution composition>

The following PCR reaction solutions were prepared as to PrimeSTAR GXL and KOD Fxneo according to their respective protocols of the products based on included buffers, dNTPs, etc.

### ■ PrimeSTAR GXL-containing PCR reaction solution

| | |
|---|---|
| Template DNA | 0.1 ng/µL |
| dNTP | 0.2 mM |
| Forward primer | 0.3 µM |
| Reverse primer | 0.3 µM |
| 5× PrimeSTAR GXL Buffer | 0.2 µL/10 µL |
| PrimeSTAR GXL DNA polymerase | 1 µL/10 µL |

### ■ KOD Fxneo-containing PCR reaction solution

| | |
|---|---|
| Template DNA | 0.1 ng/µL |
| dNTP | 0.4 mM |
| Forward primer | 0.3 µM |
| Reverse primer | 0.3 µM |
| 2× KOD FX neo buffer | 5 µL/10 µL |
| KOD FX neo DNA polymerase | 0.2 µL/10 µL |

### ■ Pfu-containing PCR reaction solution

| | |
|---|---|
| Template DNA | 0.1 ng/µL |
| dNTP | 0.4 mM |
| Forward primer | 0.5 µM |
| Reverse primer | 0.5 µM |
| Tris hydrochloride buffer solution | 0.15 M |
| DNA polymerase Pfu | 0.2 µL/10 µL |
| MgSO₄ | 2.5 mM |
| DMSO | 5% |

The PCR reaction was performed using C1000 Touch thermal cycler (manufactured by Bio-Rad Laboratories, Inc.) under the following conditions.

| | |
|---|---|
| (1) Initial thermal denaturation | 98°C for 10 seconds |
| (2) Thermal denaturation | 98°C for 10 seconds, 30 seconds, 60, or 120 seconds |
| (3) Annealing | 60°C for 10 seconds, 30 seconds, 60, or 120 seconds |
| (4) Extension reaction | 68°C for 10 seconds, 30 seconds, 60, or 120 seconds |
| (5) 30 cycles of (2) to (4) | |

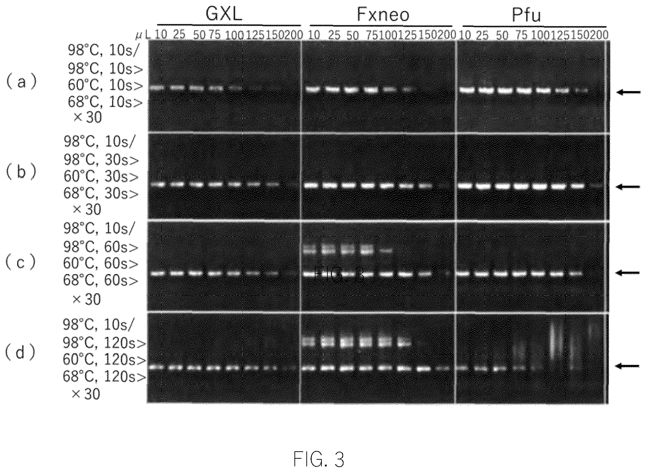

Results of examining the obtained PCR product by agarose electrophoresis are shown in Figure 3. As is evident from Figure 3, nucleic acid amplification was hardly seen for any of the enzymes when the reaction solution exceeded 150 µL. Slight amplification was seen in 200 µL by lengthening the times of thermal denaturation, annealing, and extension reaction. However, a smear band (blurry band continuously extending up and down) was seen due to the longer times of thermal denaturation, annealing, and extension reaction, confirming that nonspecific nucleic acid amplification occurred. Previous PCR widely practiced is often carried out in 50 µL at the maximum. This is probably based on the fact that amplification efficiency or specificity is reduced if the amount of the reaction solution is 50 µL or more, particularly, exceeds 150 µL.

### [Example 3] Study on pH of PCR reaction solution

Next, the pH of the PCR reaction solution was studied. A PCR reaction solution was prepared so as to have the same composition as that of the Pfu-containing PCR reaction solution of Example 2 described above except that the pH was adjusted to 7.70, 7.90, 8.10, 8.30, 8.52, 8.81, 9.26, or 9.86. The PCR reaction solution was 10 µL, and PCR reaction was performed by 5 types given below. The number of cycles was 30 cycles in all the cases.
(a) Thermal denaturation at 98°C for 2 minutes, annealing at 60°C for 2 minutes, and extension reaction at 68°C for 2 minutes
(b) Thermal denaturation at 98°C for 10 seconds, and annealing and extension reaction at 72°C for 10 seconds
(c) Thermal denaturation at 98°C for 10 seconds, and annealing and extension reaction at 72°C for 2 minutes
(d) Thermal denaturation at 98°C for 2 minutes, and annealing and extension reaction at 72°C for 10 seconds
(e) Thermal denaturation at 98°C for 2 minutes, and annealing and extension reaction at 72°C for 2 minutes

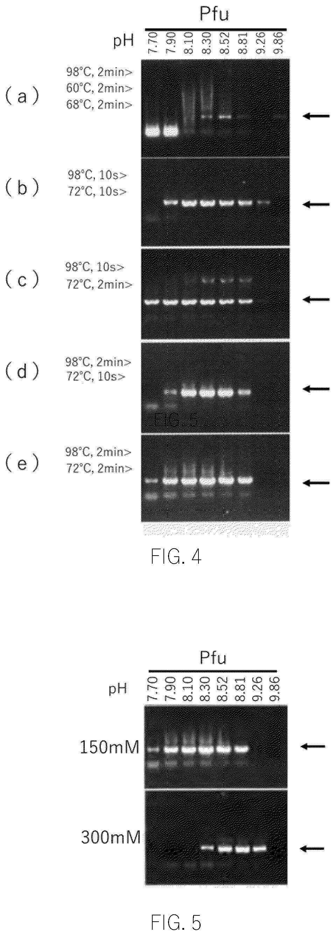

Results of agarose electrophoresis of each PCR product are shown in Figure 4. As shown in Figure 4, it was revealed that in all the cases, nucleic acid amplification efficiency was high under alkaline conditions of pH 7.9 or higher, and nucleic acid amplification efficiency was high at 72°C for annealing and extension reaction.

### [Example 4] Study on pH and buffer solution concentration of PCR reaction solution

Focusing on the buffer solution concentration of the PCR reaction solution, PCR was performed using the same Pfu-containing PCR reaction solution as in Example 2 described above except that: the concentration of the Tris hydrochloride buffer solution was set to 150 mM or 300 mM; and the same pH as in Example 3 described above was used. The PCR reaction solution was 10 µL, and PCR reaction was performed as given below. The number of cycles was 30 cycles.

### Thermal denaturation at 98°C for 2 minutes, and annealing and extension reaction at 72°C for 2 minutes

Results of agarose electrophoresis of each PCR product are shown in Figure 5. As shown in Figure 5, it was revealed that a clearer band indicating decrease in nonspecific amplification was seen with increase in the concentration of the buffer solution, and amplification efficiency was good under alkaline conditions of pH 8.3 or higher.

### [Example 5] Study on PCR reaction time and buffer solution concentration

In light of the results of Examples 3 and 4 described above, the relationship between a PCR reaction time and a buffer solution concentration was examined. PCR was performed using the same Pfu-containing PCR reaction solution as in Example 2 described above except for the concentration of the buffer solution. The PCR reaction solution was 10 µL, and PCR was performed by 8 types of reaction temperatures and times, and buffer solution concentrations shown in Table 1 below.

**[Table 1]**

| No. | | Temperature | Time |
|---|---|---|---|
| (a) | Thermal denaturation | 90°C | 10 sec |
| | Annealing and extension reaction | 65°C | 10 sec |
| (b) | Thermal denaturation | 90°C | 10 sec |
| | Annealing and extension reaction | 65°C | 10 sec |
| (c) | Thermal denaturation | 90°C | 30 sec |
| | Annealing and extension reaction | 65°C | 30 sec |
| (d) | Thermal denaturation | 90°C | 30 sec |
| | Annealing and extension reaction | 65°C | 30 sec |
| (e) | Thermal denaturation | 90°C | 60 sec |
| | Annealing and extension reaction | 65°C | 60 sec |
| (f) | Thermal denaturation | 90°C | 60 sec |
| | Annealing and extension reaction | 65°C | 60 sec |
| (g) | Thermal denaturation | 90°C | 10 sec |
| | Annealing and extension reaction | 65°C | 60 sec |
| (h) | Thermal denaturation | 65°C | 10 sec |
| | Annealing and extension reaction | 90°C | 60 sec |

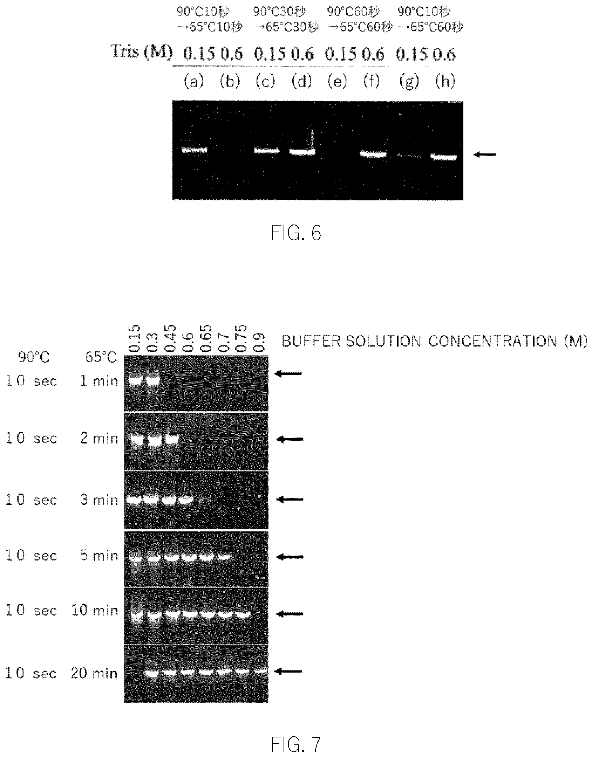

Results of agarose electrophoresis of each PCR product are shown in Figure 6. As shown in Figure 6, it was confirmed that when the concentration of the buffer solution was 0.15 M, nucleic acid amplification efficiency was markedly reduced due to the thermal denaturation time or the annealing and extension reaction time of 60 seconds as shown in (e) or (g). On the other hand, it was confirmed that when the concentration of the buffer solution was 0.6 M, nucleic acid amplification efficiency was poor due to the annealing and extension reaction time as short as 10 seconds as shown in (b) whereas nucleic acid amplification efficiency was improved by the annealing and extension reaction time of 30 seconds or longer as shown in (d), (f), and (h). When the reaction solution has a large volume, the times of annealing and extension reaction are inevitably long by temperature regulation for PCR thermal cycles, as compared with the case of an ordinary amount of a reaction solution. From the results described above, it was found that PCR in a large volume is difficult with a buffer solution concentration of 0.15 M which is a concentration currently used widely whereas the problem of a long annealing and extension reaction time associated with the large volume can be solved by increasing the concentration of the buffer solution.

### [Example 6] Study on PCR reaction time and buffer solution concentration - 2

In light of the results of Examples 3 to 5 described above, the relationship between a PCR reaction time and a buffer solution concentration was further examined. PCR was performed using the same Pfu-containing PCR reaction solution as in Example 2 except that the concentration of the Tris hydrochloride buffer solution was set to 0.15, 0.3, 0.45, 0.6, 0.65, 0.7, 0.75, or 0.9 M. The reaction solution was 10 µL, and PCR was performed by thermal denaturation at 90°C for 10 seconds, and annealing and extension reaction at 65°C for 1 minute, 2 minutes, 3 minutes, 5 minutes, 10 minutes, or 20 minutes as reaction times and reaction temperatures.

Results of agarose electrophoresis of each PCR product are shown in Figure 7. As shown in Figure 7, in the case of the 0.15 M buffer solution, nucleic acid amplification efficiency was reduced due to the annealing and extension reaction time of 5 minutes or longer, and a smear band and nonspecific amplification were further seen. No nucleic acid was amplified when the time was 20 minutes or longer. The 0.3 M or higher buffer solution permitted nucleic acid amplification even when the annealing and extension reaction time was 20 minutes or longer, and a higher concentration of the buffer solution reduced nonspecific amplification.

In the case of performing large-volume PCR, conditions adaptable to reaction for a long time are necessary from the viewpoint of temperature control. In consideration of this Example together with Examples 3 to 5 described above, it was confirmed that for performing large-volume PCR, the control of the concentration and pH of the buffer solution is very important from the viewpoint of nucleic acid amplification efficiency and specific amplification.

### [Example 7]

For study on various reaction solutions or reaction conditions for PCR reaction up to Example 6 described above, a microtube was used as a reaction container. However, the microtube cannot be adapted to a large volume of a reaction solution. Accordingly, PCR was performed using a plastic bag as a reaction container in order to perform large-volume PCR.

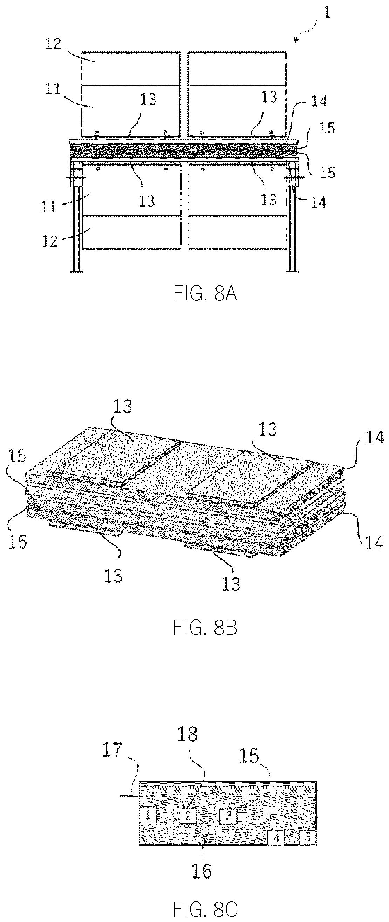

50 mL of water was added into a polypropylene autoclave bag (thickness: 35 pm, length: 22.5 cm, width: 10 cm; Violamo), which was then hermetically sealed by heat seal that blocked air from entering the bag. Two such bags were provided as models of a reaction container housing 50 mL of a PCR reaction solution. These two bags were stacked as two layers between which polypropylene autoclave bags having a thickness of 35 pm, a length of 3 cm, and a width of 3 cm and housing 200 µL of a Pfu reaction solution (hereinafter, also referred to as "200 µL reaction solution bags") were sandwiched. The resultant was loaded in a thermal cycler. PCR employed a thermal cycler 1, a modification of the thermal cycler described in patent document 4 described above, as shown in Figure 8A. Briefly speaking, a temperature regulation unit of the thermal cycler 1 is constituted by air cooling Peltier elements 13 (two each on the underside and on the upper face) having heat sinks 11 and cooling fans 12, aluminum plates 14 having a short axis of 10 cm and a long axis of 22.5 cm and adhering to the above Peltier elements 13 by screwing, and a temperature control part (not shown). The temperature control part has a current control part, an operation part, a set temperature input part, and a power supply part. As shown in Figures 8B, the above 200 µL reaction solution bags 16 were sandwiched between the above autoclave bags 15 stacked as two layers which were flanked by 3 mm thick aluminum plates 14 adhering to pairs of upper and lower Peltier elements 13. The resultant was placed in the thermal cycler 1. The 200 µL reaction solution bags 16 were sandwiched between the autoclave bags 15 such that the 200 µL reaction solution bags were disposed at five sites represented by 1 to 5 in Figures 8C on the upper face of the lower layer. A thermometry part 18 of an affixable low-profile thermocouple temperature sensor 17 (RKC Instrument Inc.) was affixed to the underside of the 200 µL reaction solution bag 16 at position No. 2. Information on the detected outside surface temperature of the 200 µL reaction solution bag was sent to the current control part, and the temperature was regulated by controlling current to the Peltier elements 13. The temperature data logger used was GL840 (Graphtec Corp.). The temperature measurement conditions involved a temperature measurement range of 0 to 500°C and a sampling interval of 0.5 seconds.

The PCR reaction was performed as follows.

| | |
|---|---|
| (1) Thermal denaturation | 90°C for 10 seconds |
| (2) Thermal denaturation | 90°C for 30 seconds |
| (3) Annealing and extension reaction | 65°C for 2.5 minutes |
| (4) 30 cycles of (2) and (3) | |

A Pfu-containing PCR reaction solution was prepared so as to have a composition (final concentration) given below. A polynucleotide encoding HGF-BGH as shown in SEQ ID No: 5 was used as template DNA instead of the polynucleotide encoding EGFP. The nucleotide sequences of a forward primer and a reverse primer are shown in SEQ ID Nos: 6 and 7, respectively. A PCR amplification product is 3167 bp as shown in SEQ ID No: 8.

### ■ Pfu-containing PCR reaction solution

| | |
|---|---|
| Template DNA (human HGF-BGH 1 ng/µL) | 0.1 ng/µL |
| dNTP | 0.4 mM |
| Forward primer | 0.5 µM |
| Reverse primer | 0.5 µM |
| Tris hydrochloride buffer solution (pH 9.23) | 0.6 M |
| Pfu DNA polymerase | 0.3 µL/10 µL |
| MgSO₄ | 2.5 mM |

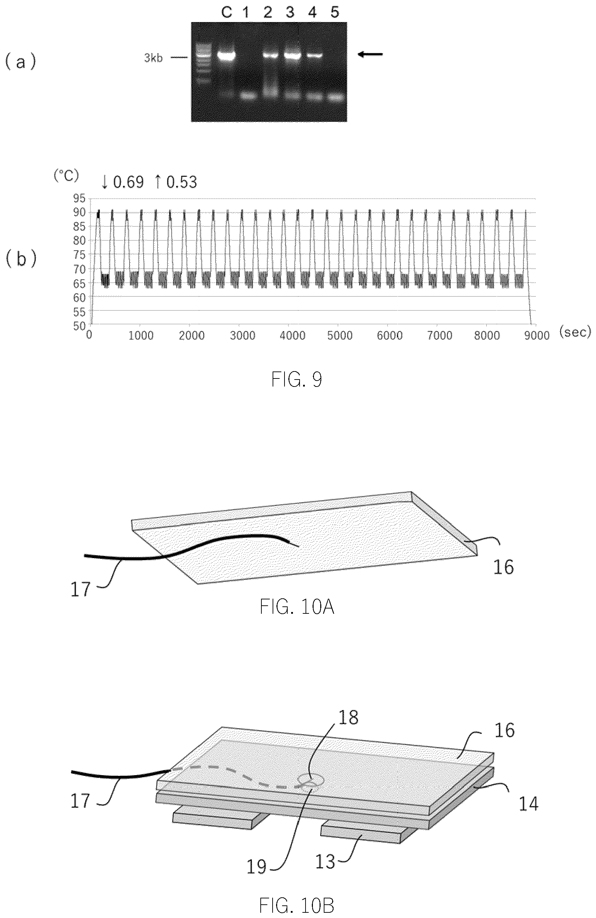

Results of agarose electrophoresis of each PCR product are shown in the upper part of Figure 9. In Figure 9, C depicts results of applying 1 µL of a PCR reaction solution when PCR was performed in a 200 µL microtube housing 10 µL of a reaction solution as a control of a conventional method, and 1 to 5 each depict results of applying 1 µL each of the PCR reaction solutions in the 200 µL reaction solution bags disposed at the positions shown in Figure 8C. As shown in Figure 9, the nucleic acid was amplified in the 200 µL reaction solution bags at positions 2 to 4 shown in Figure 8C whereas no nucleic acid was amplified in the 200 µL reaction solution bag at position 1 or 5, confirming that the inside temperatures of the reaction solution bags were uneven in thermal cycles. Results of further examining the temperatures of PCR cycles in the 200 µL reaction solution bag at position 2 shown in Figure 8C are shown in the lower part of Figure 9. In the lower part of Figure 9, the abscissa depicts a time (sec) elapsed from the start of PCR reaction (the start of warming for initial thermal denaturation), and the ordinate depicts the outside surface temperature of the reaction solution bag measured with the temperature sensor affixed to the 200 µL reaction solution bag. From in the lower part of Figure 9, the thermal denaturation temperature or the annealing and extension reaction temperature fluctuated in a range of 63 to 69°C, and the temperature was variable without maintaining the set temperature. From these results, it was considered that the temperature of a liquid in an amount as large as 50 mL fluctuated, resulting in reduction in thermal denaturation or annealing and extension reaction efficiency.

### [Example 8]

In light of Example 7 described above, it is presumably necessary to uniformize PCR reaction solutions in PCR reaction solution bags, and adjust temperatures to set temperatures at the thermal denaturation, annealing and extension reaction stages when the amount of a PCR reaction solution is further increased. Accordingly, whether or not the problem could be solved by stirring a PCR reaction solution was tested.

In Example 7, bags housing 50 mL of water were stacked as two layers. In this Example, (1) 100 mL of a PCR reaction solution was housed in one reaction solution bag, (2) 200 mL of a PCR reaction solution was housed in one reaction solution bag, or (3) 300 mL of water was housed in one reaction solution bag as a model of the case of housing 300 mL of a reaction solution while a plastic bag containing 200 µL of a PCR reaction solution was contained in the reaction solution bag, and air was put into the reaction solution bag of (1) to (3) such that its inner volume was 5 to 10%. Such a reaction solution bag was installed in the thermal cycler described in Example 7, and the thermal cycler itself was placed on a shake table of a shaker (Rotary Shaker SR-300; Shimadzu Corp.). Then, the thermal cycler was fixed to the shake table so as to convey rotary shake from the shaker to the reaction bag installed in the thermal cycler. PCR was performed while the reaction solution was stirred by stirring the reaction container through rotary shake at 100 rpm. For a control with no shake, one bag housing 50 mL of a reaction solution was installed in the thermal cycler where PCR was performed without shake. The composition of the PCR reaction solution and PCR thermal cycles were the same as in Example 7. As shown in Figure 10A, the low-profile thermocouple temperature sensor 17 was affixed to the center on the underside of the reaction solution bag 16. As shown in Figure 10B, the reaction solution bag 16 was placed on an aluminum plate 14, a heat conductive plate, such that the thermometry part 18 of the temperature sensor was disposed at the center of a through-hole 19 provided in the aluminum plate 14. In the thermal cycler, a copper plate was used instead of the aluminum plate 14 only for 300 mL of the reaction solution.

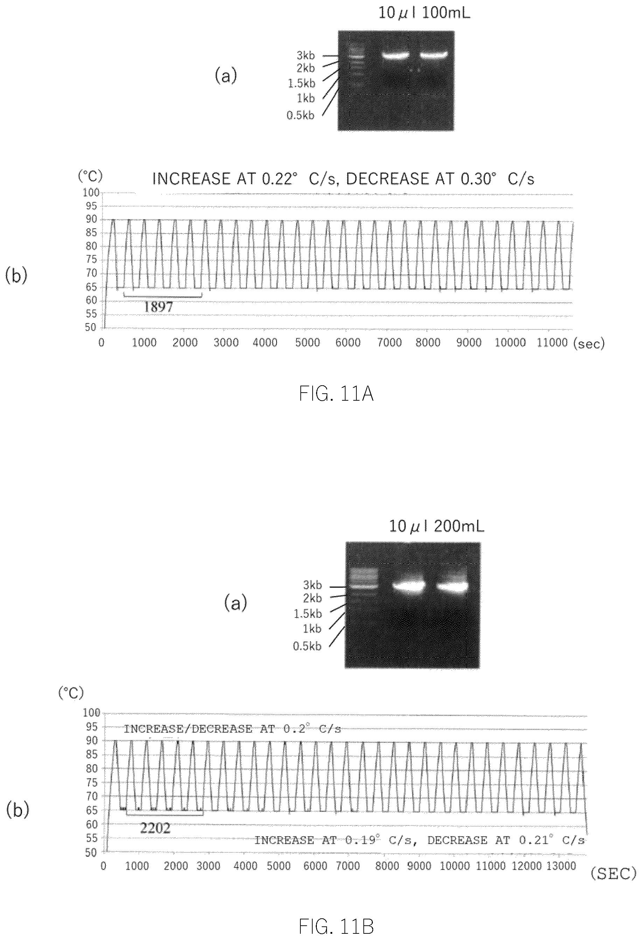

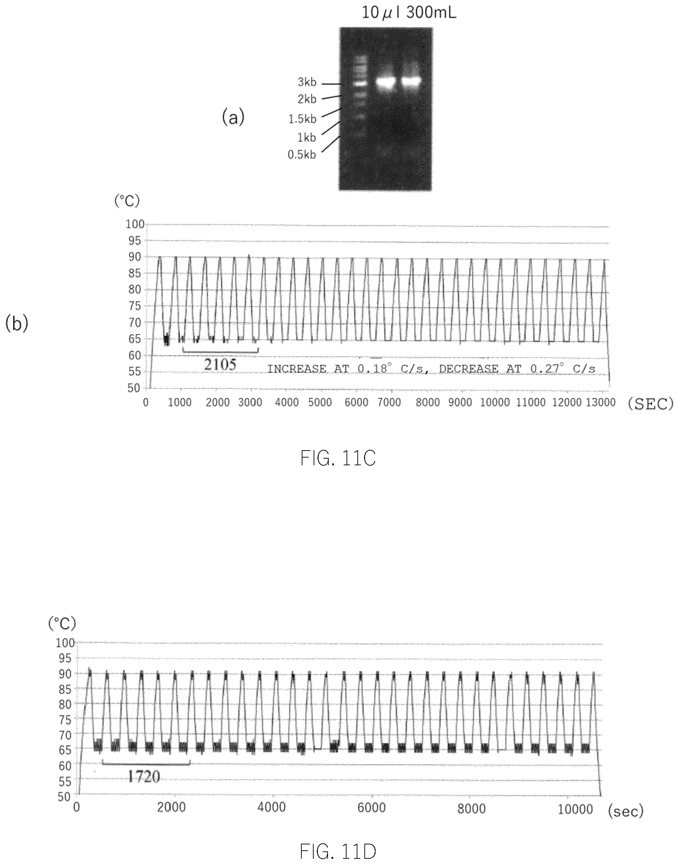

Results about 100 mL of the reaction solution are shown in Figure 11A. Results about 200 mL of the reaction solution are shown in Figure 11B. Results about 300 mL of the reaction solution are shown in Figure 11C. Results about the control with no shake are shown in Figure 11D. In Figures 11A to 11C, the left 10 µL lane was obtained as a control when PCR was performed without shake using a microtube for 200 µL housing 10 µL of a reaction solution. (a) of Figures 11A to 11C upper part shows results of agarose electrophoresis. (b) of Figures 11A to 11C lower part and Figure 11D show results of examining the temperatures of thermal cycles. The abscissa depicts a time (sec) from the start of PCR reaction, and the ordinate depicts a temperature. The time required for one reaction cycle was lengthened with increase in the volume of the reaction solution, though the reaction time and temperature were set to the same values in all the cases. The time required for 5 cycles were 1897 seconds, 2202 seconds, and 2105 seconds for 100 mL, 200 mL, and 300 mL, respectively. In spite of concerns about a long reaction time that might worsen nucleic acid amplification efficiency, it was confirmed that the nucleic acid was amplified by the stirring of the reaction solution even when the reaction solution had a volume as large as 100 to 300 mL. As for the reaction temperature, the temperature of thermal denaturation, or annealing and extension reaction was variable for the control with no shake shown in Figure 11D whereas the temperature was controlled at more nearly the set temperature for 100 mL, 200 mL, and 300 mL with shake.

### [Example 9]

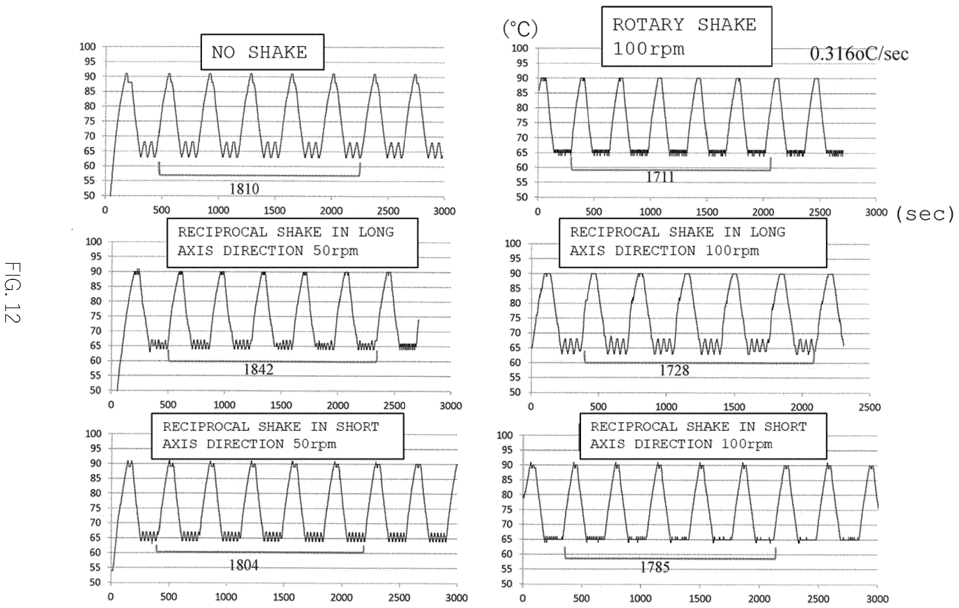

In Example 8, rotary shake was used. Other manners of shake were studied. PCR was performed in the same way as in Example 8 except that: no shake, rotary shake at 100 rpm, reciprocal shake at 50 rpm or 100 rpm in the long axis direction, or reciprocal shake at 50 rpm or 100 rpm in the short axis direction was performed; and a reaction solution bag housing 100 mL of a reaction solution was used. The results are shown in Figure 12. In Figure 12, the abscissa depicts a time (sec) from the start of PCR reaction, and the ordinate depicts a temperature. As shown in Figure 12, the time per 5 cycles was as short as 1711 seconds for rotary shake at 100 rpm. It was confirmed that the temperature was controlled at more nearly the set temperature for rotary shake at 100 rpm or short-axis shake at 100 rpm.

### [Example 10]

From Examples 8 and 9 described above, it was able to be confirmed that the temperature can be controlled to be approximately closer to the set temperature by shaking the reaction solution bag. In this context, when a bag houses 100 mL or more of a PCR reaction solution, water vapor may be generated within the reaction solution bag at the thermal denaturation stage and before or after this stage so that the reaction solution bag is swollen while air accumulates on the upper side. In this case, if a heat source is provided on the upper side of a reaction container, temperature control from the upper side is not efficient because heat is conveyed to a PCR reaction solution via air. Accordingly, whether a nucleic acid could be amplified by temperature control only on the underside of a bag housing a PCR reaction solution was tested in light of a simple structure of a thermal cycler, and reduction in the variability of the whole temperature by the stirring of the PCR reaction solution through shake.

PCR was performed by the same method as in Example 7 through rotary shake at 100 rpm using a reaction solution bag housing 200 mL of a Pfu-containing PCR reaction solution. In the thermal cycler shown in Figure 8A, the temperature control of the upper Peltier elements was stopped, and a bag housing only air (air thickness: 2 to 5 cm) was placed between the upper side of the reaction solution bag and the upper Peltier elements, for heat insulation. Specifically, heat sources were located only on the underside of the reaction solution bag. For a control, the same rotary shake at 100 rpm was performed, and temperature control was performed in both the upper Peltier elements and the lower Peltier elements. A copper plate was used as a heat conductive plate instead of the aluminum plate.

Results of measuring the temperature of PCR reaction are shown in Figures 13 upper part. Figure 13 upper left part shows results of examining PCR thermal cycles when PCR reaction was performed by temperature control only on the lower side, and Figure 13 upper right part shows results of examining PCR thermal cycles when PCR reaction was performed by temperature control both on the upper side and on the lower side. It was confirmed that even temperature control only on the lower side without temperature control on the upper side accomplished almost the same level of temperature control by rotary shake as that for the control (temperature control both on the upper side and on the underside). Results of agarose electrophoresis when PCR was performed by putting a plastic bag housing 100 µL of a reaction solution into a bag housing 200 mL, 300 mL, or 400 mL of water as a model of 200 mL, 300 mL, or 400 mL of a reaction solution, are shown in Figures 13 lower part. In the case of using 200 to 400 mL of reaction models, it was also confirmed that the nucleic acid was amplified by temperature control only on the lower side.

### [Example 11]

Since it was confirmed that nucleic acids could be amplified even at 400 mL in the above Example 10, we investigated whether nucleic acid amplification at even larger volumes was possible.

As a model for the 600 mL or 1000 mL reaction solution, PCR was performed in the same manner as in Example 10 by placing a plastic bag containing 100 µL of Pfu-containing PCR reaction solution in a 32 cm long and 32 cm wide autoclave bag (containing 5-10% volume of air) containing 600 mL or 1000 mL of water and rotating the bag at 100 rpm.

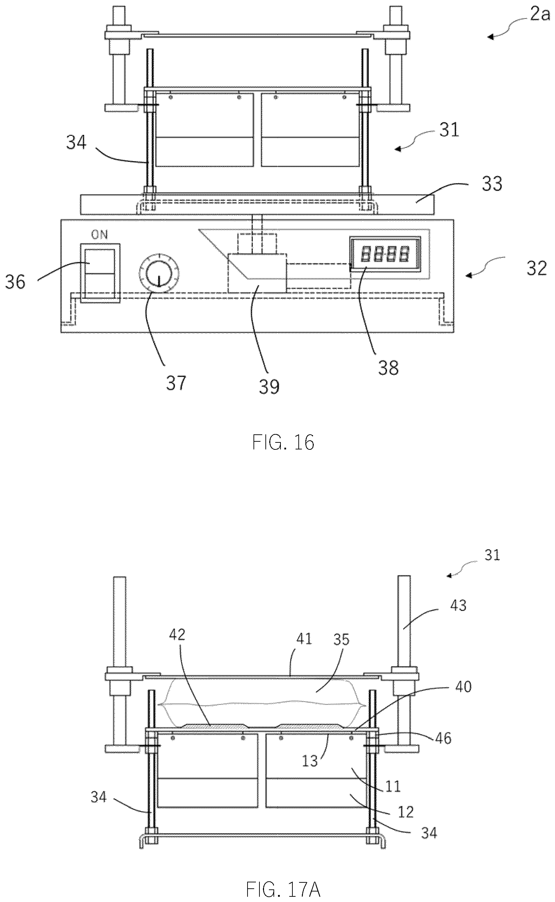

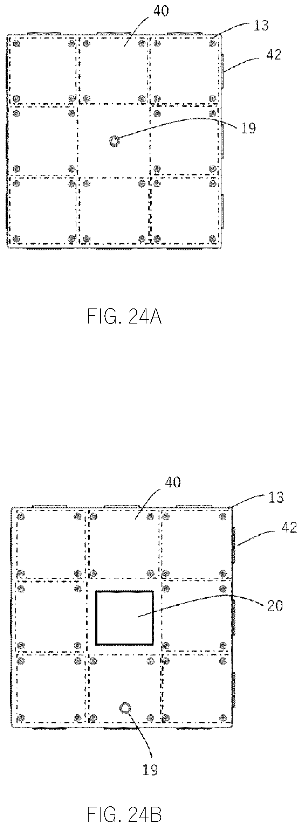

As the thermal cycler, the first example of the thermal cycler shown in Figure 16, which will be described later, was used, and a bag containing only air (2-5 cm thick of air) was placed above the reaction solution bag between it and the Peltier element to insulate it. In other words, only the bottom surface of the reaction solution bag was in contact with the heat conductive plate, which acted as a heat source. The copper plate shown in Figure 24A, which will be described later, was used as the heat conductive plate, and about eight Peltier elements were fixed to the copper plate so that they were placed in parallel on the horizontal plane in the shape of the square on the sides excluding the center portion. The concentration of Tris hydrochloric acid buffer in the reaction solution was 0.6 M or 0.65 M for the 600 mL model and 0.6, 0.65, or 0.7 M for the 1000 mL model. Furthermore, the concentration of template DNA was 0.1 ng/µL (final concentration) in the 600 mL reaction solution model and 0.1 ng/µL (final concentration) or 1 ng/µL (final concentration) in the 1000 mL reaction solution model.

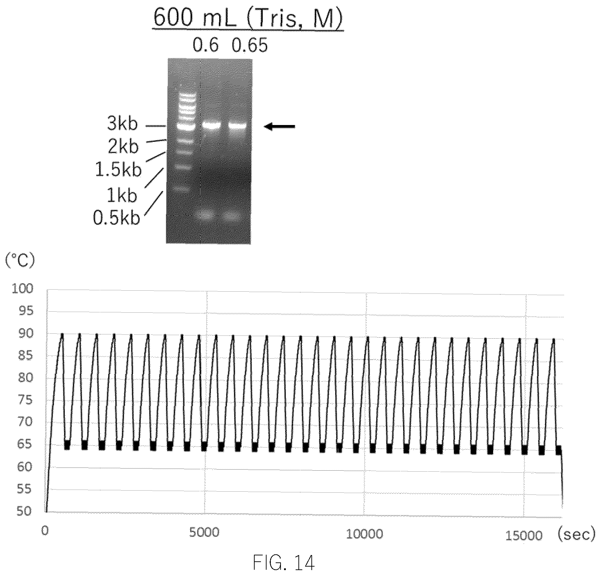

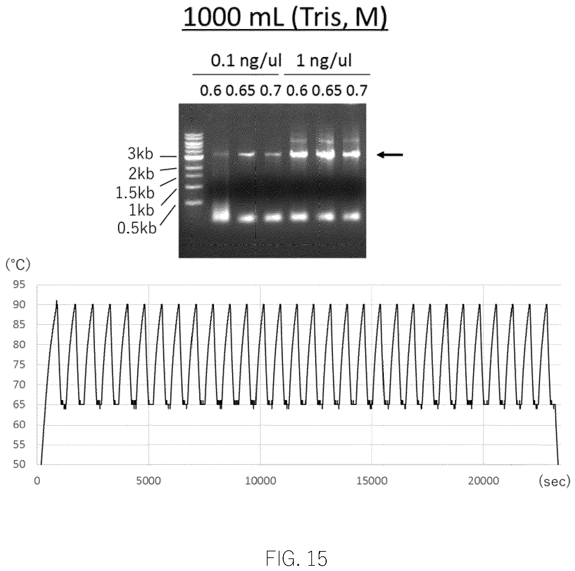

The results of agarose electrophoresis and the results of thermal cycling when PCR was performed in the 600 mL and 1000 mL models of reaction solution, respectively, are shown in Figures 14 and 15. The target nucleic acids were amplified in both models, confirming that nucleic acids can be amplified by the present method of amplifying nucleic acids even in large volumes of 600 mL or 1000 mL.

The temperature rise in the second cycle in the case of the model of 1000 mL reaction solution was 0.06°C /second and the temperature fall was 0/16°C /second. In the conventional PCR method, the temperature rise and fall are generally 1 to 6°C/second, but in the amplification method of the present invention, the amplified product was obtained even at a slow temperature rise of 0.06°C/second, which is extremely surprising for a nucleic acid amplification technology that has always required a fast temperature rise rate. As shown in the above example, by controlling the concentration and pH of the buffer solution, it was possible to amplify the target nucleic acid even if the time required for 30 cycles was 160 minutes or more, 200 minutes or more, 250 minutes or more, or 360 minutes or more.

### [Example 12]

### (First example of thermal cycler)

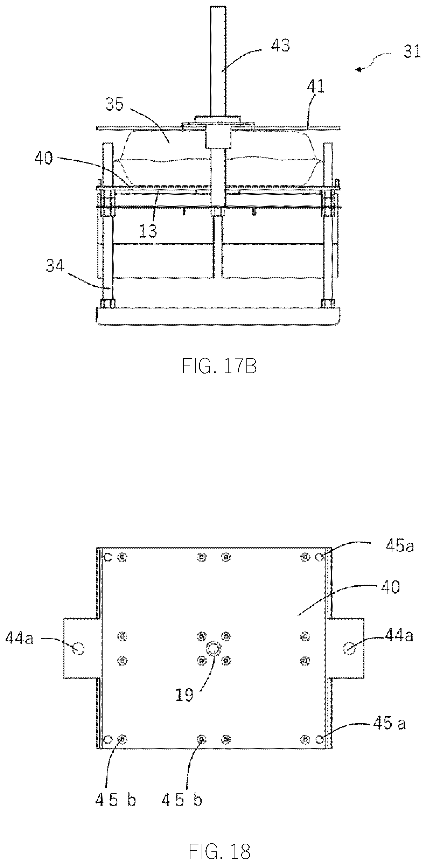

Next, a thermal cycler 2 that can be used in the method for amplifying a nucleic acid according to the present invention will be described as a first example with reference to Figures 16 and 17. Figure 16 is a front view of the whole first example of thermal cycler 2. Figure 17A is a front view of a temperature regulation unit 31. Figure 17B is a right side view of the temperature regulation unit 31. The temperature regulation unit 31 is fixed through four temperature regulation unit fixing shafts 34 having an outer circumferential spiral groove onto a shake table 33 of a shake unit 32. Owing to this fixation, a reaction container 35 rotates by the rotary shake of the shake table 33 of the shake unit 32 so that a reaction solution (not shown) in the reaction container 35 is rotary shaking.

The shake unit 32 has an on/off switch 36 of shake, a rotating speed adjustment dial 37, and a rotating speed display part 38 and can control shake by adjusting a rotating speed to a predetermined speed. A motor 39 is installed at the center of the main body of the shake unit 32 such that the rotation axis is located in the vertical direction. The motor 39 is perpendicularly joined to the shake table 33. Rotary shake is attained by the power of the motor 39.

The temperature regulation unit 31 has four Peltier elements 13 of 2 × 2 in parallel on a horizontal plane and a 3 mm thick copper plate 40 screwed such that the whole upper faces of the Peltier elements 13 are closely attached thereto. The reaction container 35 housing a PCR reaction solution is placed on the copper plate 40. The heat of the Peltier elements 13 is transferred to the reaction container 35 via the copper plate 40. A stainless-steel reaction container support plate 41 is placed under its own weight on the upper face of the reaction container 35. An anti-displacement plate 42 for preventing the displacement of the reaction container by oscillation is installed on the lateral face of the copper plate 40.

The Peltier element 13 has the heat sink 11 and the cooling fan 12 on a face opposite to the face in contact with the reaction container 35. The lower end of the cooling fan 12 is positioned at a predetermined height from the base ends of the temperature regulation unit fixing shafts 34, and a space is provided so as to prevent the convection of air released from the cooling fan 12. The Peltier element 13 is connected to a temperature control part having a power control part, an operation part, a power supply part, a temperature display part, and a set temperature input part (not shown).

Figure 18 shows a plane view pf the copper plate 40. The copper plate has the through-hole 19 of 10 mm in diameter at the center. Both ends have insertion holes 44a for the insertion of linear shafts 43 provided such that the reaction container support plate 41 is freely movable in the vertical direction. Four corners have screw holes 45a for connection to the temperature regulation unit fixing shafts 34 with screws, and four sets of four screw holes 45b for connection to the Peltier elements 13 with screws. The copper plate 40 is screwed to the Peltier elements 13 via the screw holes 45a for connection to the Peltier elements 13. As shown in Figure 17A, the temperature regulation unit fixing shafts 34 having a nut 46 as a stopper at a predetermined position are inserted into the screw holes of the copper plate 40 so that the copper plate 40 is fixed under its own weight to the temperature regulation unit fixing shafts 34.

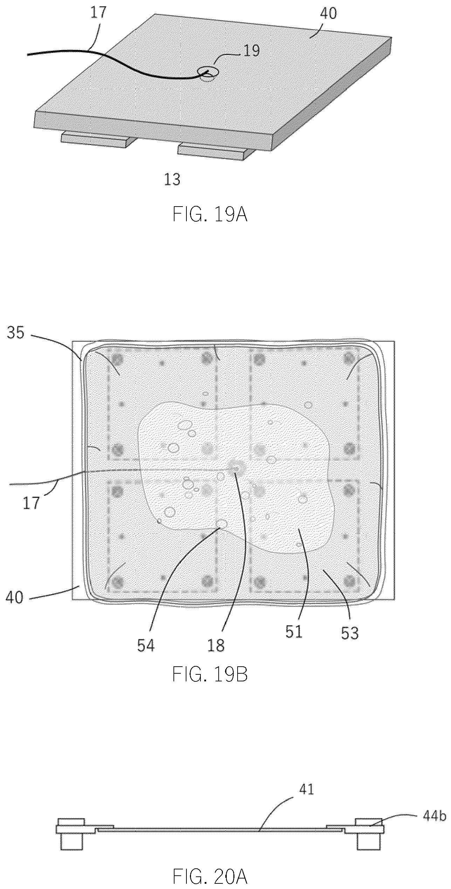

Next, the disposition of the temperature sensor will be described with reference to Figures 10A and 19A, B. The affixable low-profile thermocouple temperature sensor 17 is affixed to a central part on the outside surface of the reaction container 35. The thermometry part 18 of the temperature sensor 17 is disposed, as shown in Figure 19A, in the through-hole 19 present at the central part of the copper plate 40 so as not to come into contact with the copper plate 40 (the reaction container is not shown). Figure 19B is a plane view of a state where the reaction container 35 housing a PCR reaction solution and containing 10% of air 51 is placed on the copper plate 40 while the thermometry part 18 of the temperature sensor 17 is positioned at the center (the reaction container support plate is omitted from the drawing). Results of detecting a temperature by this temperature sensor 17 are sent to the temperature control part (not shown). Then, power to be supplied to the Peltier elements 13 from the power control part is determined in the operation part. Specifically, the temperature control part has a function of regulating power to be supplied to the Peltier elements 13 and controlling the temperature of the copper plate 40, based on temperature data on the reaction container 35 detected by the temperature sensor 17.

In the set temperature input part, information necessary for PCR thermal cycles, for example, the reaction temperatures of initial thermal denaturation, thermal denaturation, annealing, and extension reaction, reaction times, and the number of cycles, are input by an operator. The temperature measured by the temperature sensor 17 is displayed in the temperature display part.

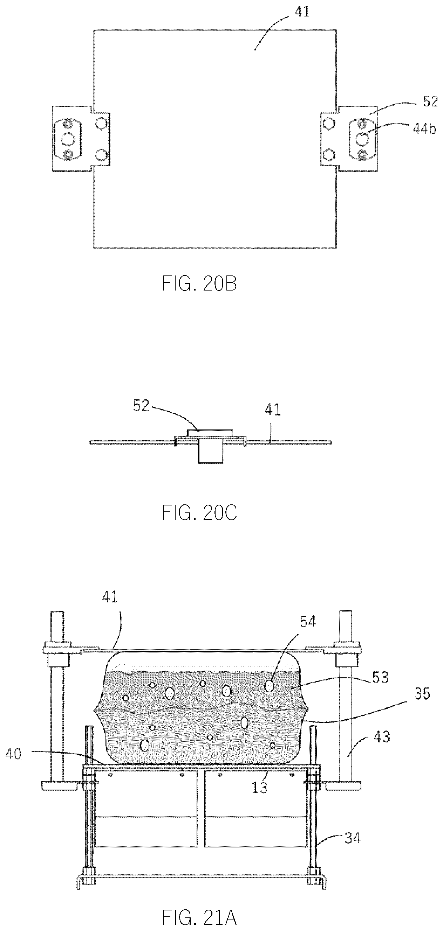

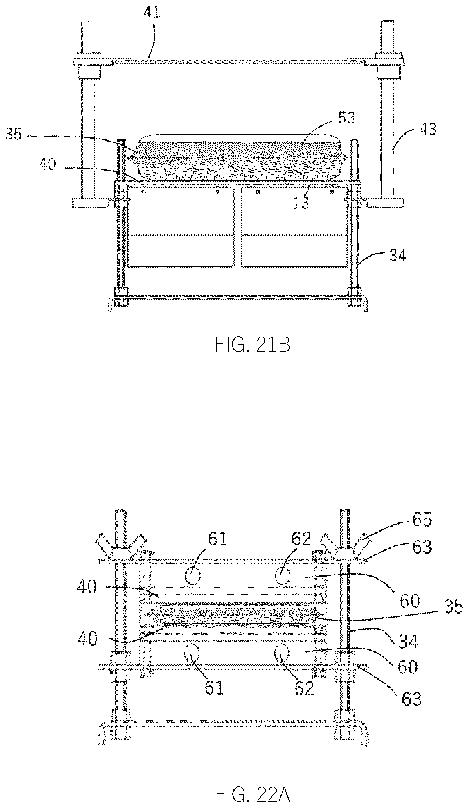

The reaction container support plate 41 will be described with reference to Figures 20A, 20B, and 20C and Figures 21A and 21B. Figures 20A, 20B, and 20C show a front view, a plane view, and a side view, respectively, of the reaction container support plate 41. Figure 21A shows a state where the reaction container 35 is swollen by air accumulating on the upper side due to water vapor 54 generated in the reaction container. Both ends of the reaction container support plate 41 have support plate holding parts 52 having insertion holes 44b for insertion of the linear shafts 43. The reaction container support plate 41 freely goes up and down in the vertical direction with the linear shafts 43 by the insertion of the linear shafts 43, and also abuts, under its own weight, the upper face of the reaction container 35. Owing to such a configuration, when the height of the upper face of the reaction container is increased due to water vapor generated from a PCR reaction solution in the reaction container or when the height of the upper face of the reaction container is decreased by decrease in the amount of water vapor in the reaction container, the reaction container can be held and supported without regulating the height of the reaction container support plate. Furthermore, with this configuration, The reaction container support plate 41 abuts the upper face of the reaction container 35 according to the height of the upper face of the reaction container 35 while close attachment between the underside of the reaction container 35 and the copper plate 40 can also be maintained. The Peltier elements 13 are supported by the temperature regulation unit fixing shafts 34, and the reaction container support plate 41 is supported by the linear shafts 43. This structure can prevent the heat of the copper plate 40 from being conducted to the reaction container support plate 41 and therefore attains more precise temperature control.

In Figure 21B, the reaction container support plate 41 is provided with fixing means that can be fixed in a position that allows a predetermined gap between it and the reaction container 35. In the process of cooling the reaction solution after thermal denaturation and for annealing, a gap is provided between the reaction container 35 and the reaction container support plate 41 to allow air to enter, as shown in Figure 21B, to improve the heat radiation efficiency of the reaction solution and speed the temperature drop.

### [Example 13]

### (Second example of thermal cycler)

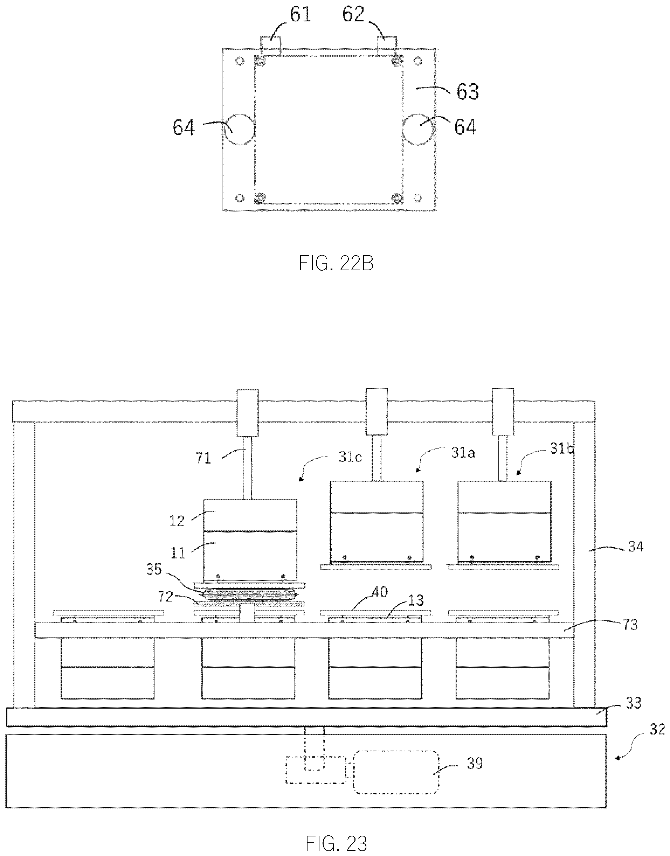

Next, a thermal cycler that can be used in the method for amplifying a nucleic acid will be described as a second example with reference to Figure 22A and 22B. The stirring unit is omitted in Figure 22a. In Example 10 described above, the Peltier elements 13 are based on air cooling by the cooling fans 12. By contrast, the Peltier elements 13 shown in the second example are based on water cooling. A pair of upper and lower cooling units based on water cooling using the Peltier elements 13 are provided (the Peltier elements 13 are not shown because of being present within cooling units 60), and solvent inlets 61 for cooling and solvent outlets 62 are provided therein. Upper copper plate 40 has a role of a reaction container support plate. Reaction container support plate fixing shafts are absent, and two cooling unit support plates 63 are provided in parallel with the temperature regulation unit fixing shafts 34. The Peltier elements 13 and the cooling units 60 are supported by the cooling unit support plates 63. Both ends of the cooling unit support plate 63 have insertion holes 64 through which the temperature regulation unit fixing shafts 34 are inserted, and are fixed to the temperature regulation unit fixing shafts 34 through thumbscrews 65 from above.

### [Example 14]

### (Third example of thermal cycler)

A thermal cycler that can be used in the method for amplifying a nucleic acid will be described as a third example with reference to Figure 23. In order to accelerate the warming and cooling rates of a reaction solution with a large volume, three temperature regulation units are provided for sets of upper and lower temperature regulation units 31: a temperature regulation unit 31a for thermal denaturation, a temperature regulation unit 31b for anneal and extension reaction, and a preheating or cooling temperature regulation unit 31c for performing temperature increase or decrease in a shorter time such that the reaction container attains the set temperature of the next step in a short time. In each of the upper temperature regulation units 31a to 31c, the air cooling Peltier element 13 having the heat sink 11 and the cooling fan 12 can go up and down in the perpendicular direction by a lifting cylinder 71. In controlling the temperature of the reaction container 35, the Peltier element goes down and abuts the upper face of the reaction container 35 to fix the reaction container 35, while enhancing heat conduction efficiency by strengthening adherence to the copper plate 40. Each of the upper temperature regulation units 31a to 31c is in a lifted state when the reaction container 35 resides in a different temperature regulation unit. The upper temperature regulation units may be mere heaters that can be controlled to a given temperature or may have no heat source.

Each of the temperature regulation units 31a to 31c has a low-profile thermocouple temperature sensor which measures the outside surface temperature on the underside of the reaction container 35, and a low-profile thermocouple temperature sensor which measures the temperature of a copper plate having a length of 20 cm, a width of 10 cm, and a thickness of 5 mm (not shown), and information on each temperature measured is sent to a current control part (not shown), followed by temperature control. In the case of this third example of the thermal cycler, overshoot or undershoot occurs easily due to rapid increase or decrease in temperature. Thus, the temperature is controlled by PID.

The temperature regulation units 31a to 31c are fixed onto the shake table 33 of a shaker having the motor 39 via the temperature regulation unit fixing shafts 34. The reaction container 35 can be oscillated by shaking the shake table 33. The reaction container 35 may be oscillated, without the use of the shake unit 32, by rocking the whole or a portion of at least one temperature regulation unit 31 in such a way that the reaction container may be sandwiched between a set of upper and lower temperature regulation units 31 so that the whole temperature regulation units or copper plates 40 are oscillated.

The reaction container 35 is placed on a moving table 72 made of a heat conductive sheet and is movable among the temperature regulation units 31a to 31c along a traveling rail 73.

Next, the movement of this third example of the thermal cycler in the case of performing thermal cycles will be described. First, the reaction container 35 is installed in the temperature regulation units 31a for thermal denaturation adjusted to the set temperature of thermal denaturation. The upper temperature regulation unit goes down by the lifting cylinder 71 so that the upper face and the underside of the reaction container 35 abuts the copper plates 40. In this state, thermal denaturation is performed for a predetermined time after the outside surface temperature of the reaction container 35 reaches the thermal denaturation temperature. Next, after completion of thermal denaturation, the upper temperature regulation unit 31a goes up, and the reaction solution is stirred by rocking the reaction container 35 through the right and left movements of the moving table 72. Then, the reaction container 35 moves to the preheating or cooling temperature regulation units 31c preset to 0°C. The upper cooling temperature regulation unit goes down to sandwich the reaction container 35 for rapid cooling of the reaction container 35. The upper temperature regulation unit 31c goes up after the outside surface of the reaction container 35 is cooled to (near) the set temperature of annealing and extension reaction. The reaction container 35 moves to the temperature regulation units 31b for anneal and extension reaction by moving the moving table 72. Then, the upper temperature regulation unit 31b for anneal and extension reaction goes down to sandwich the reaction container 35 where annealing is then performed for a predetermined time. Next, after completion of annealing and extension reaction, the upper temperature regulation unit of the temperature regulation units 31b for anneal and extension reaction goes up. The reaction container 35 moves to the temperature regulation units 31a for thermal denaturation by moving the moving table 72. Thermal denaturation is performed for a predetermined time. Rapid heating and cooling are achieved by repeating the steps in thermal cycles and enable a shortened PCR reaction time and efficient nucleic acid amplification.

### [Example 15]

### (Positions of copper plate and Peltier element)

A form of a thermal cycler that can be used in the method for amplifying a nucleic acid will be described with reference to Figure 24A wherein eight Peltier elements 13 are used on the lower face of copper plate 40. Figure 24A is a plane view of the copper plate 40 in a state where eight Peltier elements 13 (whose positions are indicated by dotted lines in the drawing) are fixed in a hollow square pattern below the copper plate 40 in the thermal cycler 2 as the first example. The copper plate 40 has a length and a breadth of 32 cm each, and a thickness of 3 mm. The copper plate 40 has a through-hole 19 with a diameter of 1 cm in the center. No Peltier element 13 is installed at the center which serves as space in which a stirrer, an ultrasonic apparatus, or the like may be installed.

Also, the area near the center may be provided with 20 hollow sections of 3 cm in length and 3 cm in width, as shown in Figure 24B. Such a configuration allows the reaction container 35 to be easily exposed to air during the step of lowering the temperature of the reaction solution, thereby improving the heat radiation efficiency. Furthermore, if the hollow section 20 is made of a mesh, grid, or linear hollow shape, it is also possible to improve the heat radiation efficiency by using the wind caused by the movement of the fan of the Peltier element 13. To improve the heat radiation efficiency, a heat pipe may be installed below the hollow section 20 to increase the cooling efficiency by the heat pipe using convection currents generated near the Peltier element 13 by the fan of the Peltier element 13. Furthermore, to improve heat radiation efficiency, the thickness of the copper plate near the center may be thinner than the peripheral area without the hollow section.

### [Example 16]

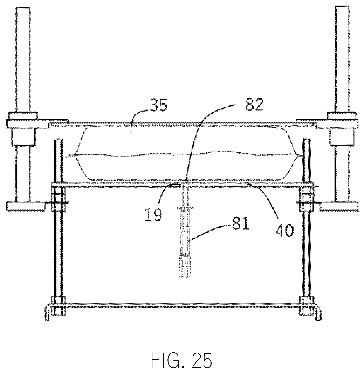

Figure 25 shows a form using a temperature probe 81 as a temperature sensor in a temperature regulation unit. Peltier elements 13, heat sinks, and cooling fans are omitted from the drawing in order to show the position of the temperature probe 81. The temperature probe 81 extensible by a spring has a thermometry part 82 on its tip. The thermometry part 82 protrudes from the through-hole 19 without coming into contact with the copper plate 40. In the case of placing the reaction container 35 on the copper plate 40, the outside surface of the reaction container 35 comes into contact with the thermometry part 82 so that the outside surface temperature of the reaction container 35 can be measured. The upper end of the thermometry part 82 need only be able to touch the reaction container 35, and depending on the material of the reaction vessel 35, it may not have to protrude through the through-hole 19.

### Industrial Applicability

The present invention enables large-volume PCR and can be utilized in the production of DNA vaccines, PCR testing, etc.

### Reference Signs List

- 1: Thermal cycler
- 2: First example of thermal cycler
- 11: Heat sink
- 12: Cooling fan
- 13: Peltier element
- 14: Aluminum plate
- 15: Autoclave bag
- 16: Reaction solution bag
- 17: Temperature sensor
- 18: Thermometry part of temperature sensor
- 19: Through-hole
- 20: Hollow section
- 31 and 31a to 31c: Temperature regulation unit
- 32: Shake unit
- 33: Shake table
- 34: Temperature regulation unit fixing shaft
- 35: Reaction container
- 36: On/off switch
- 37: Rotating speed adjustment dial
- 38: Rotating speed display part
- 39: Motor
- 40: Copper plate
- 41: Reaction container support plate
- 42: Anti-displacement plate
- 43: Linear shaft
- 44a and 44b: Insertion hole
- 45a and 45b: Screw hole
- 46: Nut
- 51: Air
- 52: Support plate holding part
- 53: PCR reaction solution
- 54: Water vapor
- 60: Cooling unit
- 61: Solvent inlet
- 62: Solvent outlet
- 63: Cooling unit support plate
- 64: Insertion hole
- 65: Thumbscrew
- 71: Lifting cylinder
- 72: Moving table
- 73: Traveling rail
- 81: Temperature probe
- 82: Thermometry part

## Claims

1. A method for amplifying a nucleic acid, comprising performing polymerase chain reaction (PCR) by controlling a temperature of one or more reaction container(s) housing a reaction solution containing a DNA polymerase, deoxyribonucleotide triphosphates (dNTPs), a template DNA, a forward primer, a reverse primer, and a buffer solution, wherein the reaction solution is in an amount of 30 mL or more, and the polymerase chain reaction is performed while the reaction solution is stirred.

2. The method according to claim 1, wherein a concentration of the buffer solution is 0.4 to 1.5 M.

3. The method according to claim 1 or 2, wherein pH of the reaction solution is 8.5 to 10.

4. The method according to any one of claims 1 to 3, wherein the reaction solution is stirred by oscillating the reaction container.

5. The method according to any one of claims 1 to 4, wherein the reaction container is made of a stretchable material.

6. The method according to any one of claims 1 to 5, wherein 1 to 90% of a gas is contained in the reaction container.

7. The method according to any one of claims 1 to 6, wherein an outside surface temperature of the reaction container or a temperature of the reaction solution is measured with a temperature sensor, and temperature control of thermal cycles in the polymerase chain reaction is performed based on the measured temperature.

8. The method according to claim 7, wherein a surface temperature of a heat source adhering to the reaction container is measured with a temperature sensor, and the temperature control of the thermal cycles in the polymerase chain reaction is performed based on the outside surface temperature of the reaction container or the temperature of the reaction solution, and the surface temperature of the heat source adhering to the reaction container.

9. A thermal cycler for use in the method of amplifying nucleic acids according to any one of claims 1 to 8, wherein the thermal cycler comprising: a temperature regulation unit which adheres to one or more reaction container(s) housing 30 mL or more of a reaction solution and regulates a temperature of the reaction solution, wherein the reaction container is made of a heat conductive material; and a stirring unit for stirring the reaction solution, wherein the temperature regulation unit comprises a heat source, a reaction container support plate which supports the reaction container by abutting an upper face of the reaction container, and a temperature sensor which measures an outside surface temperature of the reaction container or a temperature of the reaction solution.

10. The thermal cycler according to claim 9, wherein the heat source in the temperature regulation unit is a Peltier element, and the thermal cycler comprises a heat conductive plate which adheres to the Peltier element and is capable of abutting the reaction container, and a temperature control part connected to the Peltier element.

11. The thermal cycler according to claim 9 or 10, wherein temperature control of thermal cycles in polymerase chain reaction is performed based on the outside surface temperature of the reaction container or the temperature of the reaction solution measured with the temperature sensor.

12. The thermal cycler according to any one of claims 9 to 11, wherein: a heat conductive plate is provided with a depression or a through-hole; a thermometry part of the temperature sensor is disposed in the depression or the through-hole without coming into contact with the heat conductive plate; and when the reaction container is placed on the heat conductive plate, the thermometry part of the temperature sensor is capable of abutting an underside of the reaction container.

13. The thermal cycler according to any one of claims 9 to 12, wherein the thermal cycler comprises a temperature regulation unit for thermal denaturation, a temperature regulation unit for anneal and extension reaction, and a preheating or cooling temperature regulation unit.
